# EUROPEAN PATENT APPLICATION

(11) **EP 4 692 334 A1**
(43) Date of publication of application: **11.02.2026**
(21) Application number: 24780810.8
(22) Date of filing: 29.03.2024
(51) Int. Cl.: C12N 7/00, A01N 63/40, A01P 1/00, A01P 3/00, A23K 10/16, A23L 33/10, A61K 35/76, A61P 31/04, A61P 43/00, C12Q 1/70, C12N 15/33

(54) **BACTERIOPHAGE, COMPOSITION, AND METHOD FOR CONTROLLING BACTERIA BELONGING TO GENUS SALMONELLA**

(30) Priority: 31.03.2023 JP 2023057035
(71) Applicant: Kaneka Corporation, Osaka-shi, Osaka 530-8288 (JP); Rakuno Gakuen University, Ebetsu-shi, Hokkaido 069-8501 (JP); Mitsui & Co., Ltd., Chiyoda-Ku Tokyo 100-8631 (JP)
(72) Inventor: YOSHIDA, Shinichi, Iwata-shi, Shizuoka 438-0802 (JP); DOJUN, Nobuhiko, Iwata-shi, Shizuoka 438-0802 (JP); IWANO, Hidetomo, Ebetsu-shi, Hokkaido 069-8501 (JP); MURATA, Ryo, Ebetsu-shi, Hokkaido 069-8501 (JP); UCHIDA, Ikuo, Ebetsu-shi, Hokkaido 069-8501 (JP); HATANAKA, Yoshifumi, Tokyo 100-8631 (JP)
(74) Representative: Hoffmann Eitle
(86) International application number: PCT/JP2024/013049
(87) International publication number: WO 2024/204715

(57) **Abstract**

An aim of the present invention is to provide a bacteriophage having a wide host range for bacteria of the genus *Salmonella.* The present invention is a bacteriophage with bacteriolytic activity against bacteria of the genus *Salmonella,* in which the bacteriophage has a genomic DNA sequence comprising a gene encoding a tail tip protein consisting of a specific amino acid sequence.

## Description

### Technical Field

The present invention relates to a bacteriophage, a composition comprising the same, and a method for controlling bacteria of the genus *Salmonella* using the same.

### Background Art

Bacteria of the genus *Salmonella* are one of the major pathogenic bacteria of food poisoning, infect an animal, such as a human and/or a domestic animal, and cause salmonellosis, such as diarrhea. Bacteria of the genus *Salmonella* exist in a digestive tract of an animal, such as a human and/or a domestic animal, and are comprised in feces when excreted, thus causing contamination. In many cases, infection with bacteria of the genus *Salmonella* is caused by ingesting food, drink, or feed contaminated with bacteria of the genus *Salmonella.*

Conventionally, as an antimicrobial agent against bacteria of the genus *Salmonella*, a small-molecule compound has been used, but continuous use of such a compound has a negative influence, such as emergence of multidrug resistant bacteria, and thus, a new control means has been sought. A bacteriophage is highly target-specific, thus does not damage microbiota, has low virulence, and hence, has been attracting attention as a new control means against bacteria of the genus *Salmonella* in recent years (Non-Patent Literature 1).

A bacteriophage (herein often abbreviated simply as a "phage") is a generic term for viruses that infect only bacteria. Many phages attach to target host bacteria, then inject their own DNA into the bacteria, and self-amplify utilizing the translational mechanism of the bacteria. Furthermore, the bacteria are bacteriolyzed, and consequently, the amplified phages are propagated, and an infection into new target bacteria is repeated (Non-Patent Literature 2).

An example of a report on a phage bacteriolytic to bacteria of the genus *Salmonella* is described, for example, in Patent Literature 1 and 2. A phage that bacteriolyzes bacteria of the genus *Salmonella* can be used, for example, for control of bacteria of the genus *Salmonella* in chicken farming and pig farming and for detection and/or control of bacteria of the genus *Salmonella* in the field of the food industry (Non-Patent Literature 3). Practically, articles of manufacture comprising a phage bacteriolytic to bacteria of the genus *Salmonella* are already on the market (Non-Patent Literature 4), examples of which articles include: BAFASAL^{R} (Proteon Pharmaceuticals S.A.), which is a feed additive for preventing infection with bacteria of the genus *Salmonella* in a fowl; and SalmoFresh^{™} (Intralytix, Inc.) and PhageGuard (Micreos BV), which are food processing formulations that kills bacteria of the genus *Salmonella* in food.

### Citation List

### [Patent Literature]

[Patent Literature 1] WO2013-027146
[Patent Literature 2] JP2014-217336A

### [Non-Patent Literature]

[Non-Patent Literature 1] Jun-Hyun Oh et al., 2017, J. Microbiol. Biotechnol., 27(12), 2075-2088
[Non-Patent Literature 2] Sharma S. et al., Folia Microbiol., 2017, 62:17-55
[Non-Patent Literature 3] Shuai Wei et al., Microorganisms, 2019, 7, 570
[Non-Patent Literature 4] Katarzyna Zbikowska et al., Animals, 2020, 10, 872

### Summary of Invention

### Technical Problem

As described above, there is a demand for an exploration for a phage that targets bacteria of the genus *Salmonella,* from the viewpoint of development of a bacteriolytic composition for bacteria of the genus *Salmonella.*

However, frequent use of a specific phage presumably causes generation of a bacterium of the genus *Salmonella* having resistance to said phage. Thus, there is still a demand for a discovery of a new phage.

Additionally, one of the characteristics desired for a phage usable for a bacteriolytic composition is, for example, the wideness of a host range for bacteria of the *Salmonella.* A phage having a wide host range can be applied to various plant diseases, widens the range of application, and thus, is desirable.

In view of this, an object of the present disclosure is to provide a bacteriophage having a wide host range for bacteria of the genus *Salmonella.*

### Solution to Problem

The present inventors have isolated novel phages from natural dirty water and soil, using a method for detecting a bacteriolytic plaque formed on a soft agar medium having bacteria of the genus *Salmonella* cultured thereon, have evaluated the bacteriolytic activity of the phage against various bacteria of the genus *Salmonella,* and have analyzed the genomic sequence of the phage. The result of this has revealed that specific 3 bacteriophages have a wide range of bacteriolytic activity against bacteria of the genus *Salmonella,* specifically bacteriolytic activity against S. Enteritidis, S. Typhimurium, S. Infantis, S. Montevideo, and S. Javiana. The present invention has been completed on the basis of the above-described results of research and development, and specifically provides the following examples of aspects.
(1) A bacteriophage with bacteriolytic activity against bacteria of the genus *Salmonella,* wherein the bacteriophage has a genomic DNA comprising a gene encoding a tail tip protein having an ability to recognize target bacteria, and consisting of the amino acid sequence of any one of the following (a) to (c):
   (a) the amino acid sequence of SEQ ID NO: 8;
   (b) an amino acid sequence having addition, deletion, and/or substitution of one or a plurality of amino acids in the amino acid sequence of SEQ ID NO: 8; and
   (c) an amino acid sequence having a sequence identity of 99% or more to the amino acid sequence of SEQ ID NO: 8.
(2) The bacteriophage according to (1), wherein the gene encoding the tail tip protein comprises the nucleotide sequence of any one of the following (d) to (f):
   (d) the nucleotide sequence of SEQ ID NO: 9;
   (e) a nucleotide sequence having addition, deletion, and/or substitution of one or a plurality of nucleotides in the nucleotide sequence of SEQ ID NO: 9; and
   (f) a nucleotide sequence having a sequence identity of 95% or more to the nucleotide sequence of SEQ ID NO: 9.
(3) The bacteriophage according to (1) or (2), wherein the genomic DNA sequence comprises the nucleotide sequence of any one of the following (g) to (k):
   (g) the nucleotide sequence of any one of SEQ ID NOs: 10 to 12;
   (h) a nucleotide sequence having addition, deletion, and/or substitution of one or a plurality of nucleotides in the nucleotide sequence other than the nucleotide sequence of the gene within the nucleotide sequence of any one of SEQ ID NOs: 10 to 12;
   (i) a nucleotide sequence having a sequence identity of 80% or more to the nucleotide sequence other than the nucleotide sequence of the gene within the nucleotide sequence of any one of SEQ ID NOs: 10 to 12;
   (j) a nucleotide sequence having addition, deletion, and/or substitution of one or a plurality of nucleotides in the nucleotide sequence of any one of SEQ ID NOs: 10 to 12; and
   (k) a nucleotide sequence having a sequence identity of 90% or more to the nucleotide sequence of any one of SEQ ID NOs: 10 to 12.
(4) The bacteriophage according to any one of (1) to (3), wherein the bacteria of the genus *Salmonella* are S. Enteritidis, S. Typhimurium, S. Infantis, S. Montevideo, and S. Javiana.
(5) A composition comprising the bacteriophage according to any one of (1) to (4).
(6) The composition according to (5), for controlling at least S. Enteritidis, S. Typhimurium, S. Infantis, S. Montevideo, and S. Javiana.
(7) The composition according to (5) or (6), wherein the composition is a pharmaceutical composition.
(8) The composition according to (5) or (6), wherein the composition is a food/drink additive, a feed additive, or a drinking-water additive.
(9) The composition according to (5) or (6), wherein the composition is food, drink, or feed.
(10) The composition according to (5) or (6), wherein the composition is a cleaning agent, a disinfectant, a bactericide, or a sanitizer.
(11) The composition according to any one of (5) to (10), further comprising another bacteriophage(s) with bacteriolytic activity against bacteria of the genus *Salmonella.*
(12) A method for controlling bacteria of the genus *Salmonella,* comprising a contacting step of contacting the bacteriophage according to any one of (1) to (4) or the composition according to any one of (5) to (11) to a subject of application.
(13) A method for treating or preventing an infection caused by bacteria of the genus *Salmonella* in a subject, comprising an administering step of administering the bacteriophage according to any one of (1) to (4) or the composition according to any one of (5) to (11) to the subject.
(14) A method for identifying a bacterium of the genus *Salmonella,* comprising:
   a culturing step of culturing a subject bacterium isolated from a specimen suspected of comprising bacteria of the genus *Salmonella* to obtain a culture preparation;
   a mixing step of mixing the culture preparation with the bacteriophage according to any one of (1) to (4) to obtain a mixture;
   a mixture culturing step of culturing the mixture under predetermined conditions; and
   a determining step of determining that the subject bacterium is a bacterium of the genus *Salmonella,* when the subject bacterium is bacteriolyzed after the mixture culturing step.
(15) The method according to (14), wherein, in the mixture culturing step, the mixture further comprises a soft agar containing liquid medium, and the mixture is cultured on a solid medium.
(16) The method according to (14) or (15), wherein, in the culturing step, the culture preparation comprises a soft agar containing liquid medium, and the culture preparation is cultured on a solid medium.
(17) The method according to any one of (14) to (16), further comprising, before the culturing step, an isolating step of isolating the subject bacterium from the specimen suspected of comprising the bacteria of the genus *Salmonella.*

The present specification encompasses the disclosure of Japanese Patent Application No. 2023-057035 that serves as a basis for the priority of the present application.

### Advantageous Effects of Invention

The present invention can provide a bacteriophage having a wide host range for bacteria of the genus *Salmonella.*

### Brief Description of Drawings

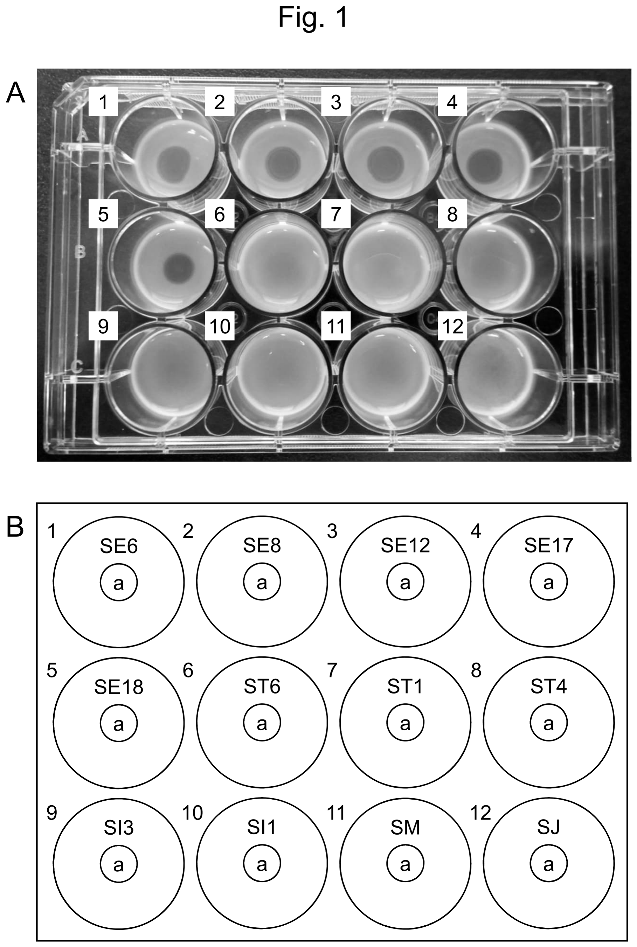

[Figure 1] Figure 1 shows the bacteriolytic activity of the 1st bacteriophage obtained in Example 1. Figure 1A shows a photograph of agar plates after culture, the agar plates obtained by spreading bacteria of the genus *Salmonella* therein, dropping a refined solution of the 1st phage thereon, and allowing the bacteria and phage to be cultured statically. Figure 1B is a diagram of the plates corresponding to Figure 1A, and shows the strain IDs of the bacteria of the genus *Salmonella* spread in the plates and the positions of the refined solutions of phage dropped onto the respective plates. In Figure 1B, "a" denotes the position of the refined solution of the phage having the genomic DNA sequence of SEQ ID NO: 7.

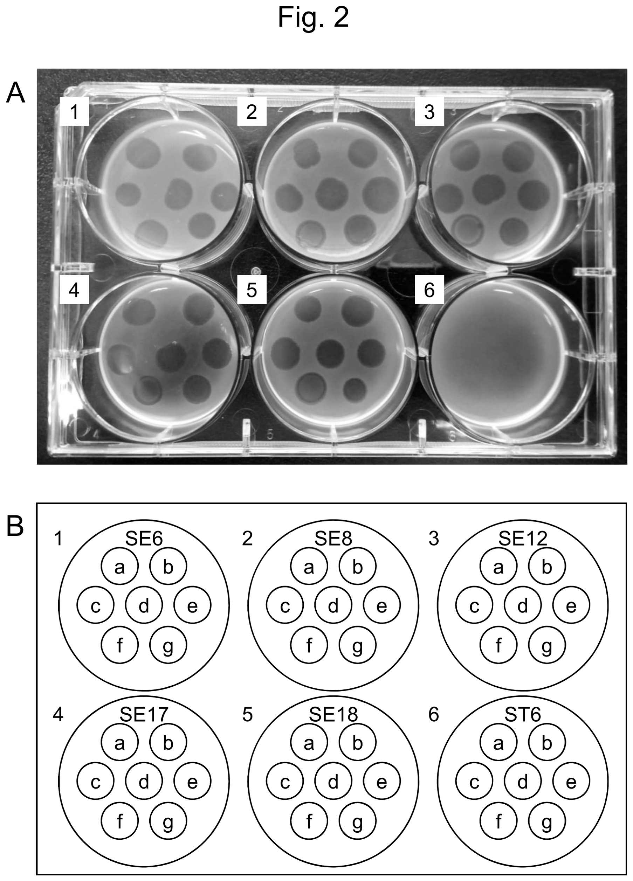

[Figure 2] Figure 2 shows the bacteriolytic activity of the 1st bacteriophage obtained in Example 1. Figure 2A shows a photograph of agar plates after culture, the agar plates obtained by spreading bacteria of the genus *Salmonella* therein, dropping a refined solution of the 1st phage thereon, and allowing the bacteria and phage to be cultured statically. Figure 2B is a diagram of the plates corresponding to Figure 2A, and shows the strain IDs of the bacteria of the genus *Salmonella* spread in the plates and the positions of the refined solutions of phage dropped onto the respective plates. In Figure 2B, "a", "b", "c", "d", "e", "f", and "g" denote the positions of the refined solutions of the phages having the genomic DNA sequences of SEQ ID NO: 1, 2, 3, 4, 5, 6, and 7 respectively.

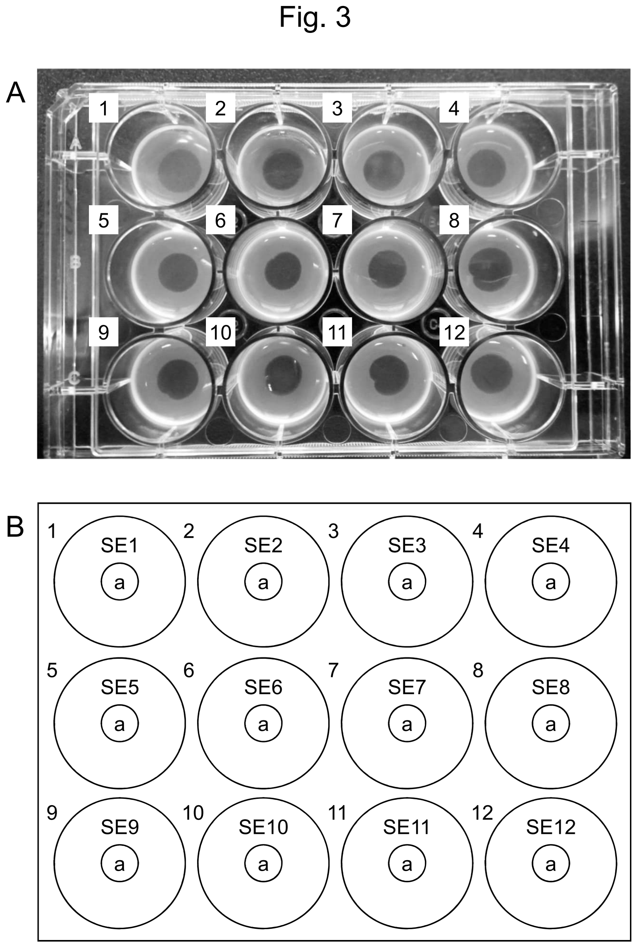

[Figure 3] Figure 3 shows the bacteriolytic activity of the 2nd bacteriophage obtained in Example 2. Figure 3A shows a photograph of agar plates after culture, the agar plates obtained by spreading bacteria of the genus *Salmonella* therein, dropping refined solutions of the 2nd phage thereon, and allowing the bacteria and phage to be cultured statically. Figure 3B is a diagram of the plates corresponding to Figure 3A, and shows the strain IDs of the bacteria of the genus *Salmonella* spread in the plates and the positions of the refined solutions of phage dropped onto the respective plates. In Figure 3B, "a" denotes the position of the refined solution of the phage having the genomic DNA sequence of SEQ ID NO: 10.

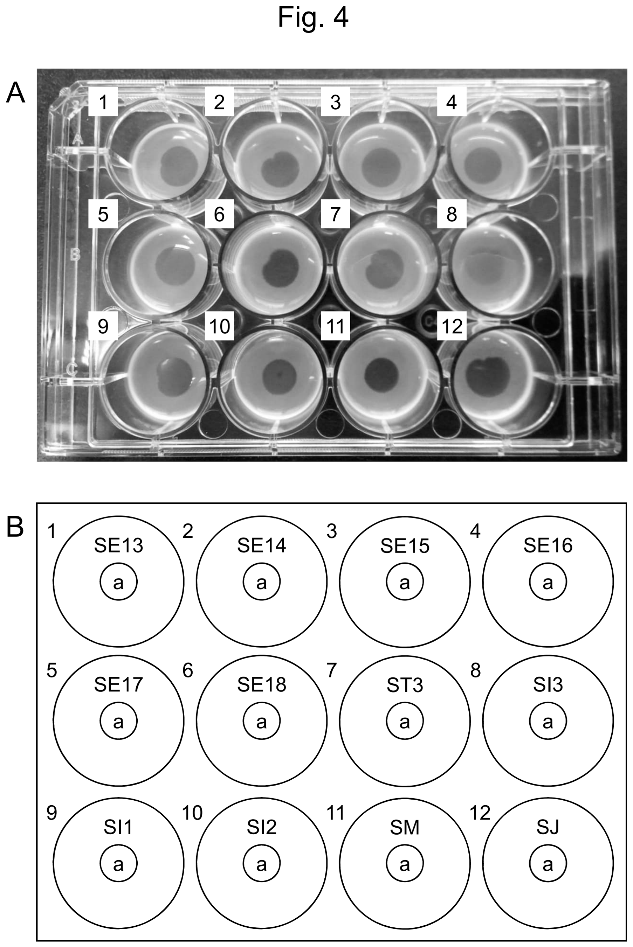

[Figure 4] Subsequent to Figure 3, Figure 4 shows the bacteriolytic activity of the 2nd bacteriophage obtained in Example 2.

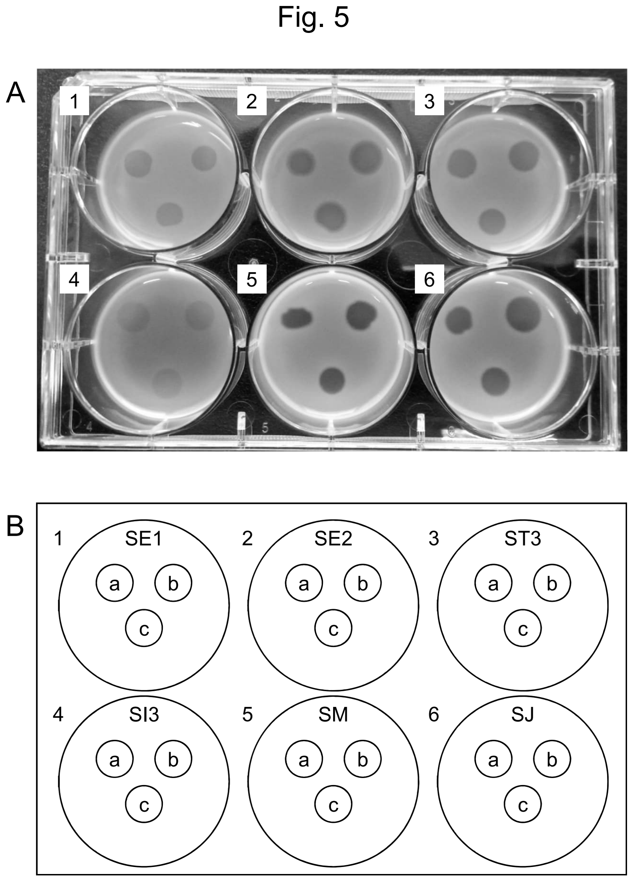

[Figure 5] Figure 5 shows the bacteriolytic activity of the 2nd bacteriophage obtained in Example 2. Figure 5A shows a photograph of agar plates after culture, the agar plates obtained by spreading bacteria of the genus *Salmonella* therein, dropping refined solutions of the 2nd phage thereon, and allowing the bacteria and phage to be cultured statically. Figure 5B is a diagram of the plates corresponding to Figure 5A, and shows the strain IDs of the bacteria of the genus *Salmonella* spread in the plates and the positions of the refined solutions of phage dropped onto the respective plates. In Figure 5B, "a", "b", and "c" denote the positions of the refined solutions of the phages having the genomic DNA sequences of SEQ ID NO: 10, 11, and 12 respectively.

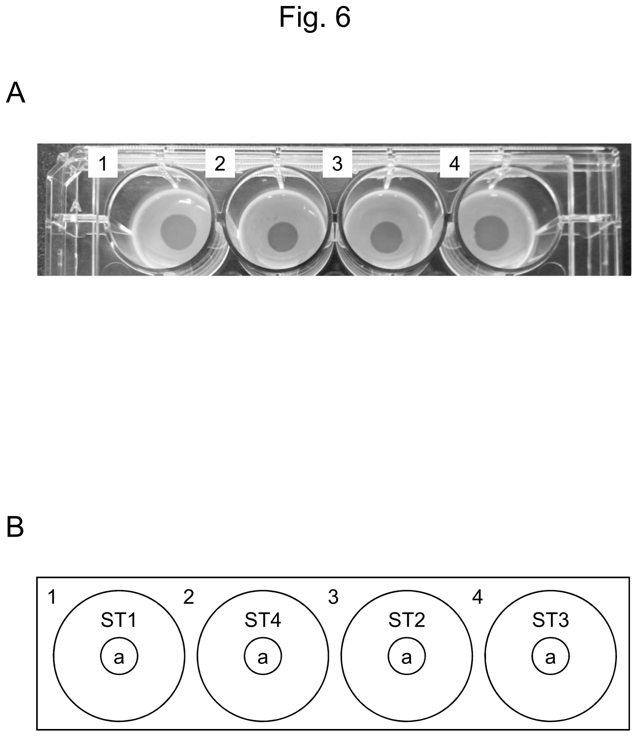

[Figure 6] Figure 6 shows the bacteriolytic activity of the 3rd bacteriophage obtained in Example 3. Figure 6A shows a photograph of agar plates after culture, the agar plates obtained by spreading bacteria of the genus *Salmonella* (*S.* Typhimurium) therein, dropping refined solutions of the 3rd phage thereon, and allowing the bacteria and phage to be cultured statically. Figure 6B is a diagram of the plates corresponding to Figure 6A, and shows the strain IDs of the bacteria of the genus *Salmonella* (*S.* Typhimurium) spread in the plates and the positions of the refined solutions of phage dropped onto the respective plates. In Figure 6B, "a" denotes the position of the refined solution of the phage having the genomic DNA sequence of SEQ ID NO: 13.

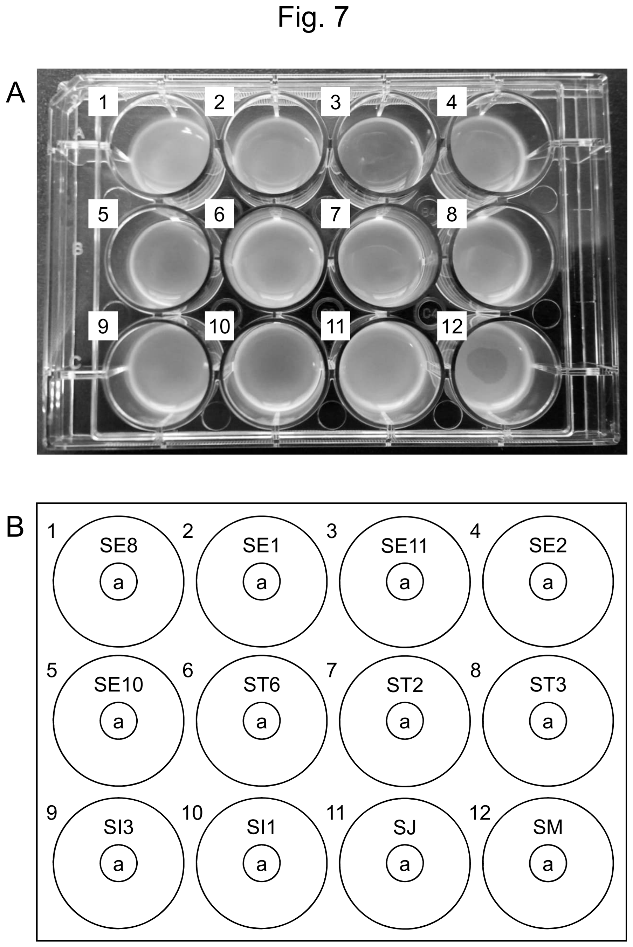

[Figure 7] Figure 7 shows the bacteriolytic activity of the 4th bacteriophage obtained in Example 4. Figure 7A shows a photograph of agar plates after culture, the agar plates obtained by spreading bacteria of the genus *Salmonella* therein, dropping a refined solution of the 4th phage thereon, and allowing the bacteria and phage to be cultured statically. Figure 7B is a diagram of the plates corresponding to Figure 7A, and shows the strain IDs of the bacteria of the genus *Salmonella* spread in the plates and the positions of the refined solutions of phage dropped onto the respective plates. In Figure 7B, "a" denotes the position of the refined solution of the phage having the genomic DNA sequence of SEQ ID NO: 14.

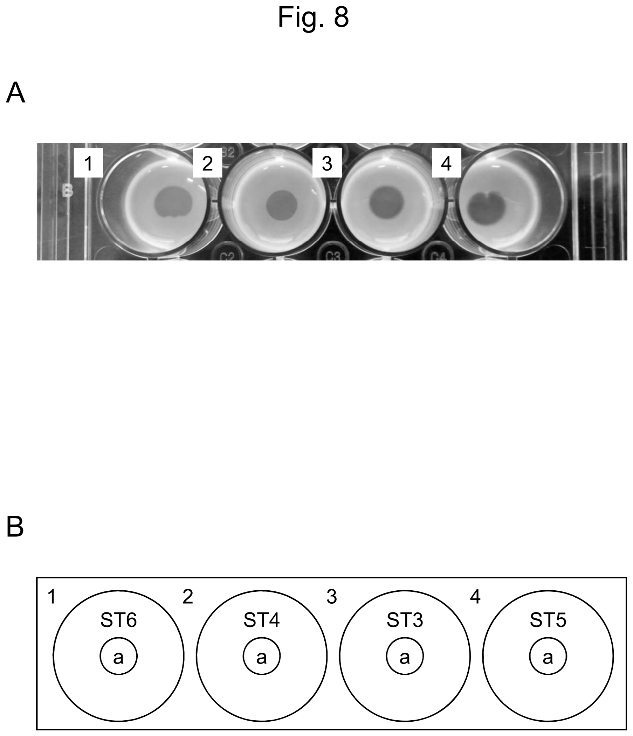

[Figure 8] Figure 8 shows the bacteriolytic activity of the 5th bacteriophage obtained in Example 5. Figure 8A shows a photograph of agar plates after culture, the agar plates obtained by spreading bacteria of the genus *Salmonella* therein, dropping a refined solution of the 1st phage thereon, and allowing the bacteria and phage to be cultured statically. Figure 8B is a diagram of the plates corresponding to Figure 8A, and shows the strain IDs of the bacteria of the genus *Salmonella* spread in the plates and the positions of the refined solutions of phage dropped onto the respective plates. In Figure 8B, "a" denotes the position of the refined solution of the phage having the genomic DNA sequence of SEQ ID NO: 17.

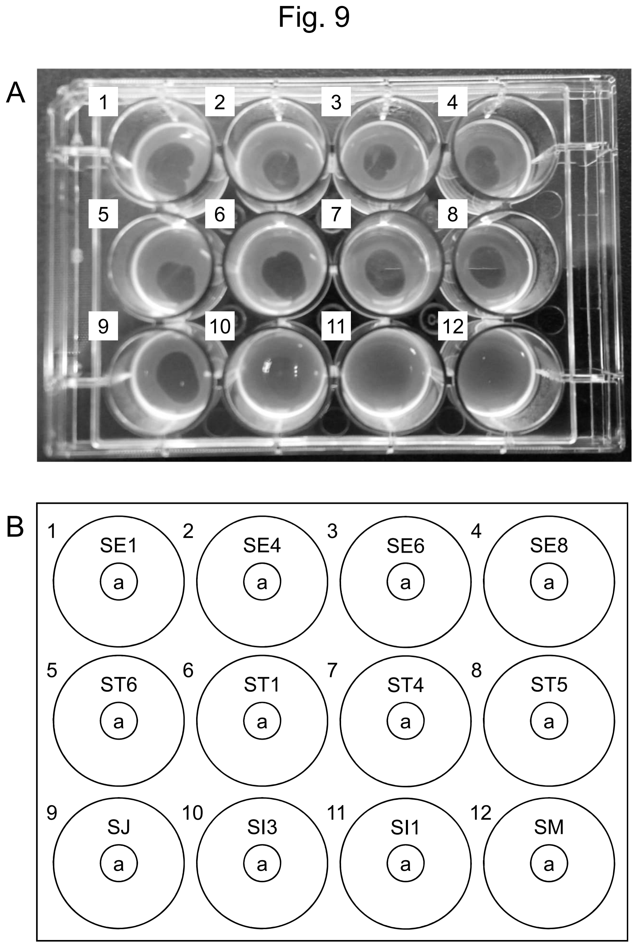

[Figure 9] Figure 9 shows the bacteriolytic activity of the 6th bacteriophage obtained in Example 6. Figure 9A shows a photograph of agar plates after culture, the agar plates obtained by spreading bacteria of the genus *Salmonella* therein, dropping a refined solution of the 6th phage thereon, and allowing the bacteria and phage to be cultured statically. Figure 9B is a diagram of the plates corresponding to Figure 9A, and shows the strain IDs of the bacteria of the genus *Salmonella* spread in the plates and the positions of the refined solutions of phage dropped onto the respective plates. In Figure 9B, "a" denotes the position of the refined solution of the phage having the genomic DNA sequence of SEQ ID NO: 20.

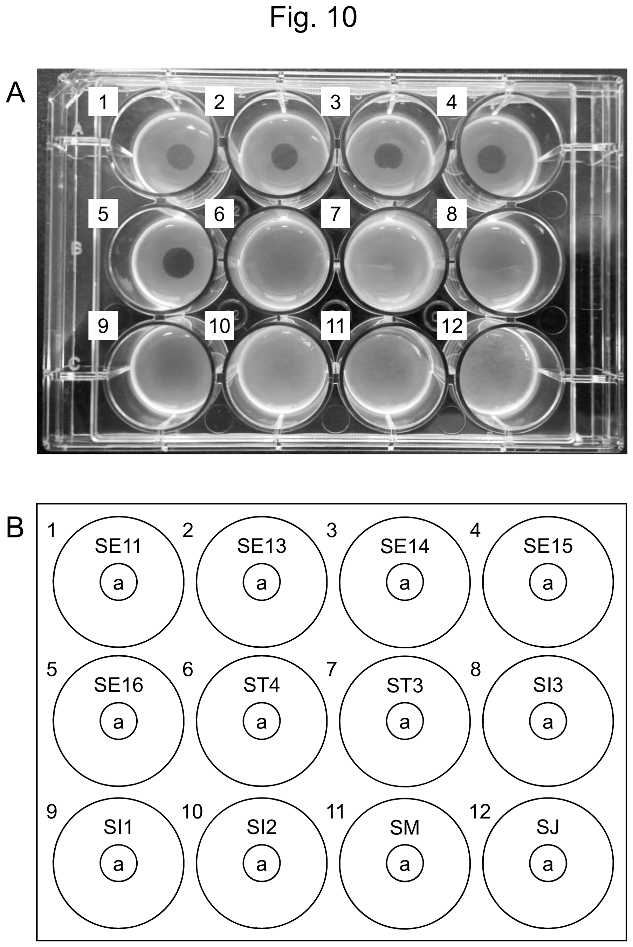

[Figure 10] Figure 10 shows the bacteriolytic activity of the 7th bacteriophage obtained in Example 7. Figure 10A shows a photograph of agar plates after culture, the agar plates obtained by spreading bacteria of the genus *Salmonella* therein, dropping a refined solution of the 7th phage thereon, and allowing the bacteria and phage to be cultured statically. Figure 10B is a diagram of the plates corresponding to Figure 10A, and shows the strain IDs of the bacteria of the genus *Salmonella* spread in the plates and the positions of the refined solutions of phage dropped onto the respective plates. In Figure 10B, "a" denotes the position of the refined solution of the phage having the genomic DNA sequence of SEQ ID NO: 23.

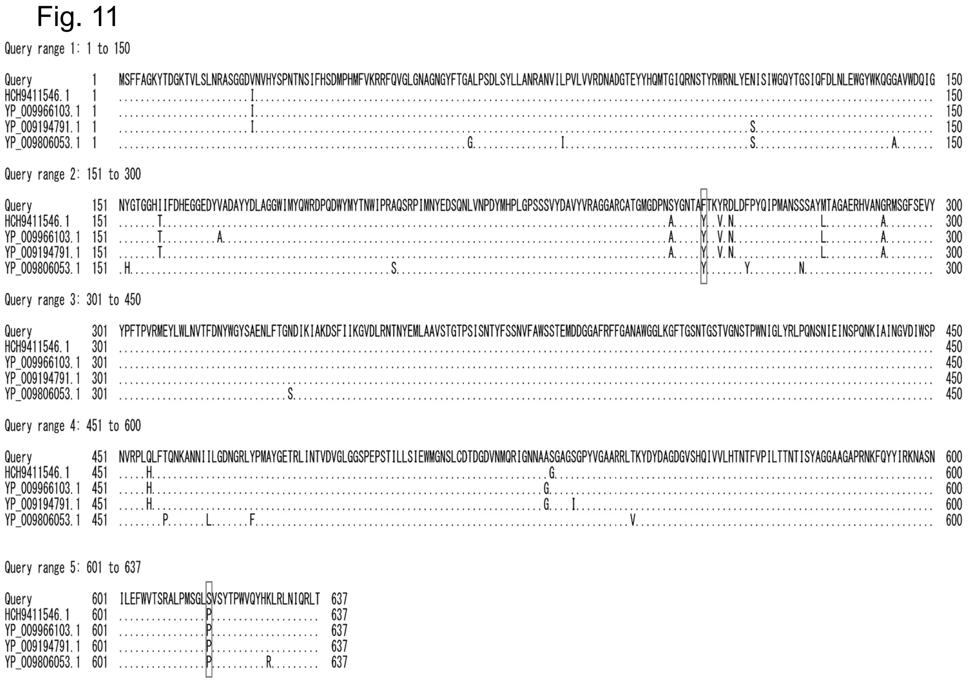

[Figure 11] Figure 11 shows the alignment between the query sequence (the amino acid sequence (SEQ ID NO: 8) of the tail tip protein of the 2nd phage obtained) and the search sequences in Example 2.

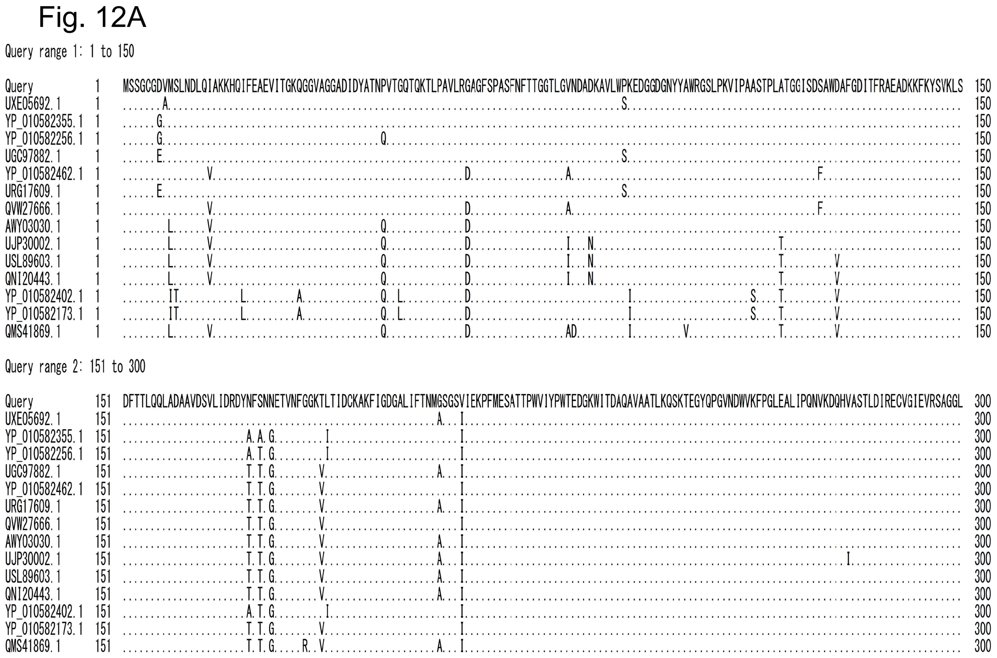

[Figure 12A] Figure 12A shows a multiple alignment performed in Example 6.

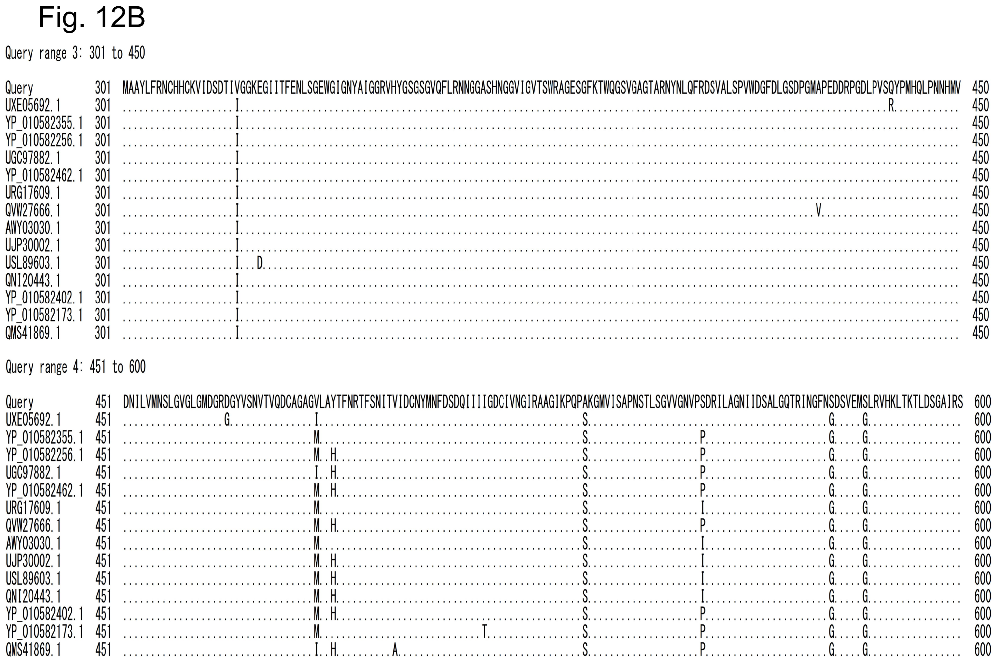

[Figure 12B] Following Figure 12A, Figure 12B shows the multiple alignment performed in Example 6.

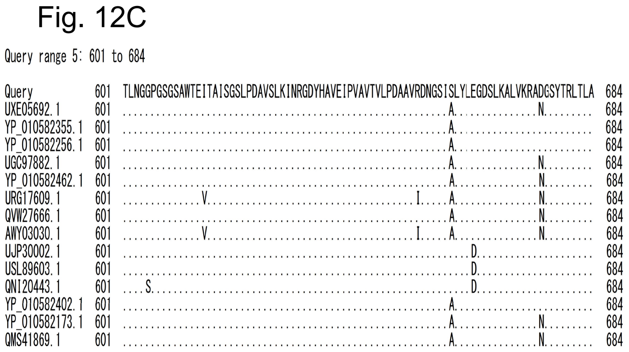

[Figure 12C] Following Figure 12B, Figure 12C shows the multiple alignment performed in Example 6.

### Description of Embodiments

The present invention will be described in detail below.

### 1. Bacteriophage

### 1-1. Overview

A first aspect of the present invention is a bacteriophage bacteriolytic to bacteria of the genus *Salmonella.* The bacteriophage of the present invention can exhibit bacteriolytic activity against S. Enteritidis, S. Typhimurium, S. Infantis, S. Montevideo, and S. Javiana. The bacteriophage of the present invention has a genomic DNA sequence comprising a gene encoding a tail tip protein consisting of a specific amino acid sequence.

The bacteriophage of the present invention can bacteriolyze and control bacteria of the genus *Salmonella* as target bacteria.

### 1-2. Definition

Terms used herein are defined below.

As used herein, "bacteriolysis" refers to a phenomenon that destroys the cell membrane of bacteria. Bacteriolysis kills bacteria. Bacteriolysis starts when a phage specifically attaches to a target bacterium and injects its own DNA into a cell of the target bacterium via the tail. Then, the phage utilizes the translational mechanism of the bacteria for self-replication to produce a large number of daughter phages and then bacteriolyzes the bacteria to release the daughter phages to the outer space.

"Bacteriolytic agent" as used herein refers to a drug comprising a bacteriophage having bacteriolytic activity against target bacteria. A bacteriolytic agent may be a bacteriolytic agent for specifically bacteriolyzing target bacteria (a target-bacteria-specific bacteriolytic agent). A bacteriolytic agent may be a bacteriophage itself.

As used herein, "bacteria", besides archaea and eukaryotes, is one of the three major biological lineages into which the whole biological world is classified. A bacterium consists of a cell without a nucleus (acaryote) and can self-replicate in the presence of a nutrient source.

"Target bacteria" as used herein refers to host bacteria that may be a target for a phage constituting the bacteriolytic agent of the present invention or a phage comprised in the composition of the present invention. A specific example is a bacterium having a membrane surface receptor on the outer membrane to be recognized by the phage. Another example is a bacterium that has, on the outer membrane, a membrane surface receptor to be recognized by a tail fiber protein, tail tip protein, tail spike protein, or tail tubular protein that consists of a specific amino acid sequence. "Membrane surface receptor" is a site to which, for example, a tail, a tail fiber, and the like of a phage are bound, and is composed of a protein, a lipopolysaccharide, pili, or the like that is present in the outer layer of the bacterial outer membrane. Target bacteria herein are, in particular, bacteria of the *Salmonella.*

As used herein, "bacteria of the genus *Salmonella*" refer to bacteria that belong to the genus *Salmonella.* Bacteria of the genus *Salmonella* are classified into 2 bacterial species: *Salmonella enterica* and *Salmonella bongori.* The former is further classified into 6 subspecies: ssp. *Enterica,* ssp. *Salamae*, ssp. *Arizonae*, ssp. *Diarizonae*, ssp. *Houtenae*, and ssp. *Indica.* Bacteria of the genus *Salmonella* are also classified into serotypes according to 2 kinds of surface structures: a somatic antigen (also referred to as an O-antigen) and a flagella antigen (also referred to as an H-antigen). The name of subspecies and serotype of bacteria of the genus *Salmonella* are expressed by a bacterial name followed by a subspecies (ssp.) and a serover (or serotype) respectively. In some cases, the name of a bacterium of the genus *Salmonella* is abbreviated as *S.* followed by a serotype. For example, *S. enterica* ssp. *Enterica* serovar Typhimurium is abbreviated as *S.* Typhimurium in some cases. The smallest unit of classification is a strain, which refers to a cell population considered to be genetically uniform.

Specific examples of serotypes of bacteria of the genus *Salmonella* include S. Enteritidis (*Salmonella enterica* ssp. *Enterica* serovar Enteritidis), *S.* Typhimurium (*Salmonella enterica* ssp. *Enterica* serovar Typhimurium), *S.* Newport, *S.* Javiana (*Salmonella enterica* ssp. *Enterica* serovar Javiana), *S.* Heidelberg, *S.* Infantis (*Salmonella enterica* ssp. *Enterica* serovar Infantis), *S.* Saintpaul, *S.* Muenchen, *S.* Montevideo (*Salmonella enterica* ssp. *Enterica* serovar Montevideo), *S.* Braenderup, *S.* Oranienburg, *S.* Thompson, *S.* Mississippi, *S.* Agona, *S.* Typhi, *S*. Bareilly, *S.* Paratyphi B, *S.* Poona, *S.* Berta, *S.* Abony, *S.* Anatum, *S.* Baildon, *S.* Bredeney, *S.* Chester, *S.* Gaminara, *S.* Hartford, *S.* Kentucky, *S.* Kiambu, *S.* Mbandaka, *S.* Nchanga, *S.* Reading, *S.* Senftenberg, *S.* Stanley, *S.* Virchow, and *S.* Urbana.

As used herein, "*Salmonella* bacteriolytic agent" refers to a bacteriolytic agent for bacteriolyzing bacteria of the genus *Salmonella.* In the same manner, *"S.* Enteritidis bacteriolytic agent" refers to a bacteriolytic agent for bacteriolyzing *S.* Enteritidis. An *S.* Enteritidis bacteriolytic agent may be a bacteriolytic agent for bacteriolyzing *S.* Enteritidis specifically (*S.* Enteritidis-specific bacteriolytic agent). *"S.* Montevideo bacteriolytic agent" refers to a bacteriolytic agent for bacteriolyzing *S.* Montevideo. An *S.* Montevideo bacteriolytic agent may be a bacteriolytic agent for bacteriolyzing *S.* Montevideo specifically (*S.* Montevideo-specific bacteriolytic agent). *"S.* Typhimurium bacteriolytic agent" refers to a bacteriolytic agent for bacteriolyzing *S.* Typhimurium. An *S.* Typhimurium bacteriolytic agent may be a bacteriolytic agent for bacteriolyzing *S.* Typhimurium specifically (*S.* Typhimurium-specific bacteriolytic agent).

As used herein, "control" of bacteria means killing bacteria and/or inhibiting proliferation of bacteria.

As used herein, "multidrug resistance" means exerting resistance to a plurality of antimicrobial agents (for example, antibiotics). Examples of antimicrobial agents include, but are not particularly limited to, ampicillin, chloramphenicol, streptomycin, sulfonamides, tetracycline, kanamycin, sulfamethoxazole/trimethoprim, cefazolin, cefotaxime, nalidixic acid, or gentamycin.

As used herein, "bacteriophage" (herein often abbreviated simply as a "phage," as mentioned above) is a generic term for viruses that infect bacteria. A common phage is composed of three parts: a head (head part), a tail (tail part), and a tail fiber (tail fiber part). The head, constituted by a capsomere that is a coat protein, is composed of a capsid (viral shell) having an icosahedral structure, in the inner space of which the genomic DNA of a phage is encapsulated. The tail has a tubular structure composed of a tail tubular protein and a sheath protein covering the same. One end and the other end of the tail are linked to the head and the tail fiber, respectively. The tail functions as an introduction tube through which the genomic DNA in the head is injected into the cell of the host bacteria. The tail fiber is composed of a structure of several fibers comprising a tail fiber protein. The tail and the tail fiber serve a host-recognizing function and an attachment function, i.e., to recognize a receptor on the surface of the outer membrane of host bacteria and be attached to the cell surface. A phage has an extremely high host specificity, and this characteristic is based on the functions of the tail and the tail fiber. More specifically, any protein of the below-described tail fiber protein, tail tubular protein, tail tip protein, and tail spike protein plays a role central to the function.

As used herein, "tail fiber protein" is a protein constituting the tail fiber of a phage, as described above. It is known that the tail fiber protein plays an important role in the specificity of the host recognition and attachment capability of the tail and the tail fiber (Nobrega F.L. et al., Nat. Rev. Microbiol., 2018, 16: 760-773). Even if the host bacteria for the novel phage characterized by the tail fiber protein are the same as for a known phage, the novel phage is different in the recognition site of the host. Thus, the novel phage may exhibit bacteriolytic activity even to bacteria having, for example, infection resistance to a known phage, thereby, its utility is very high.

As used herein, "tail fiber gene" refers to a gene encoding the tail fiber protein comprised in the genomic DNA of a phage.

As used herein, "tail tubular protein" is a protein constituting the tubular structure of the tail of a phage, as described above. It is known that the tail tubular protein interacts with the tail fiber and, together with the tail fiber, plays an important role in the specificity of the host recognition and attachment capability (Maozhi Hu, *et al.*, 2020, *9*: 1, 855-867). As tail tubular proteins, a tail tubular fiber protein A and a tail tubular protein B are known. The "tail tubular protein A" is a protein that forms a ring at the lower part of the tubular structure of the tail and interacts with the tail fiber. The "tail tubular protein B" is a protein that forms the end of the lower part of the tubular structure of the tail and binds to a receptor on the surface of the outer membrane of host bacteria.

As used herein, "tail tubular gene" refers to a gene encoding the tail tubular protein comprised in the genomic DNA of a phage. The "tail tubular protein A gene" and the "tail tubular protein B gene" refer to a gene encoding the tail tubular protein A and a gene encoding the tail tubular protein B, respectively.

As used herein, "tail tip protein" refers to a protein constituting the tip of tail part of a phage, has a sharp structure to thereby play a role in penetrating a cell wall of a host bacterium, and also has the function of binding to a receptor of the host bacterium, as described above. It is known that a tail tip protein has the function of binding to a receptor of a host bacterium, and thus, plays an important role in host recognition and attachment capability (Nobrega F.L. et al., Nat. Rev. Microbiol., 2018, 16: 760-773).

As used herein, "tail tip gene" refers to a gene encoding the tail tip protein comprised in the genomic DNA of a phage.

As used herein, "tail spike protein" refers to a protein constituting the tip of the tail part of a phage, and has the function of binding to a receptor of the host bacterium, as described above. A tail spike protein forms a spike-like structure on the bottom of a plate-like structure (tail plate) when such a plate-like structure exists at the tip of the tail part of a phage. It is known that a tail spike protein has the function of binding to a receptor of a host bacterium, and thus, plays an important role in host recognition and attachment capability (Nobrega F.L. et al., Nat. Rev. Microbiol., 2018, 16: 760-773).

As used herein, "tail spike gene" refers to a gene encoding the tail spike protein comprised in the genomic DNA of a phage.

It should be noted that a phage does not always have all of the above-described tail fiber gene, tail tubular gene, tail tip gene, and tail spike gene. A phage may comprise 1, 2, 3, or all 4 of a tail fiber gene, tail tubular gene, tail tip gene, and tail spike gene.

As used herein, "endonuclease" refers to an enzyme that cleaves a polynucleotide strand in the polynucleotide strand. It is known that, when a bacteriolytic phage infects a host bacterium, the bacteriolytic phage simultaneously takes over the life support mechanism of the host bacterium by various means, makes only the phage's self-replication possible, and shuts down the replication of the host genome at the same time. The details of the shutdown mechanism have not been clarified yet, but it has been clarified long ago that the degradation of a host genome by a nuclease derived from a phage participates in the shutdown mechanism (Warren et. Al., Journal of Virology, Vol. 2, No. 4, 1968). Accordingly, an endonuclease of a bacteriolytic phage conceivably participates in the mechanism of shutdown of the replication of a host genome.

A phage does not infect a eukaryote, and thus, a drug comprising a phage is harmless to humans, animals, and plants. In this connection, the life cycle of a phage is roughly classified into "bacteriolytic cycle", "lysogenic cycle", and "bacteriolytic/lysogenic cycle". In the lysogenic cycle, a phage incorporates its own DNA into a chromosome of target bacteria without bacteriolyzing the bacteria and proliferates as the bacteria proliferate. On the other hand, in the bacteriolytic cycle, a phage self-proliferates in a cell of host bacteria and then bacteriolyzes the host bacteria to release a large number of daughter phages. The phage of the present invention may be a phage that undergoes the bacteriolytic cycle or thebacteriolytic/lysogenic cycle.

"A plurality" as used herein refers to 2 to 10, for example, 2 to 7, 2 to 5, 2 to 4, or 2 to 3.

"Nucleotide sequence identity" as used herein is a value that indicates the ratio of the sites at which the kinds of nucleotides are the same in a range of comparison between two nucleotide sequences. Even when the two nucleotide sequences are different in length, the nucleotide sequence identity can be calculated by aligning the nucleotides so that the degree of coincidence of the nucleotides in a range of comparison becomes the highest. Without limitation, a typical algorithm for such an analysis is BLAST. BLAST may be utilized with various software or Web services. A nucleotide sequence identity may be calculated easily, utilizing, for example, the genetic information processing software GENETYX (https://www.genetyx.co.jp/), the NCBI-provided BLAST server (https://blast.ncbi.nlm.nih.gov/Blast.cgi) or the like. Besides BLAST, an algorithm called FASTA, or the like, may be utilized, as long as it is capable of calculating an appropriate identity. Additionally, a nucleotide sequence identity may be analyzed using an analysis algorithm such as MUMmer. Additionally, the software and analysis server may indicate a sequence identity according to the index, such as Average Nucleotide Identity (ANI), and these may also be used. Note that, in some of the cases where a very long nucleotide sequence such as of the genomic DNA of a phage is aligned using the above-described software or Web service, a range of comparison is determined automatically, and the sequence identity in said range of comparison is calculated. Accordingly, the above-described sequence identity may be calculated for the range to be aligned automatically provided by the above-mentioned software or Web service. For example, in some of the cases where an analysis is made using the NCBI-provided BLAST server, a query sequence and a subject sequence are aligned automatically within the maximum possible range of alignment, a range of comparison is determined, the sequence identity in the range of comparison is calculated, and also the ratio of the range of comparison to the whole range of the query sequence is calculated as a value called Query Cover. In such a case, the result can also be used as a basis for estimation of the sequence identity in the whole range of the nucleotide sequences aligned. For example, a value obtained by multiplying a Query Cover value by the value of a sequence identity in the range of comparison may be used as the estimated value of the sequence identity in the whole range. In this case, to increase the accuracy of the estimated value, a further correction may be added. For example, a sequence identity expected for a range other than the range to be aligned may be reckoned in. It should be noted that the genomic DNA of a phage is packaged linearly or circularly. Additionally, in a next-generation genome sequencer analysis, the genomic DNA is fragmented, the nucleotide sequence of each of the fragments is read, and an analysis for connecting the sequences is performed to determine a sequence. In the case of a phage, the sequences are often connected without a reference genomic DNA sequence (de novo assembly). Therefore, it is difficult to unambiguously determine the start and end of a genome to be analyzed (Merrill, B.D., et al., BMC Genomics, 2016, 17, 679). Accordingly, the starts and ends of genomic sequences in comparison may differ and are automatically taken into consideration in an analysis using software or an analysis server.

"Highly stringent conditions" as used herein refer to environmental conditions that are hardly prone to nonspecific hybridization. Under highly stringent conditions, a hybrid can be formed with a nucleic acid having a target nucleotide sequence, but a hybrid cannot substantially be formed with a nucleic acid having a nonspecific nucleotide sequence. Generally, highly stringent conditions refer to low-salt-concentration and high-temperature conditions. The low-salt concentration is, for example, 15 to 750 mM, preferably 15 to 500 mM, 15 to 300 mM, or 15 to 200 mM. Additionally, the high temperature is, for example, 50 to 68°C or 55 to 70°C. Specific examples of highly stringent conditions include conditions where hybridization is followed by washing with 0.1× SSC and 0.1% SDS at 65°C.

"Amino acid sequence identity" as used herein is a value that indicates the ratio of the sites at which the kinds of amino acid residues are the same in a range of comparison between two amino acid sequences. Even when the two amino acid sequences are different in length, the amino acid sequence identity can be calculated by aligning the amino acids so that the degree of coincidence of the amino acids in a range of comparison becomes the highest. Without limitation, a typical algorithm for such an analysis is BLAST. BLAST may be utilized with various software or Web services. An amino acid sequence identity may be calculated easily, utilizing, for example, the genetic information processing software GENETYX (https://www.genetyx.co.jp/), the NCBI-provided BLAST server (https://blast.ncbi.nlm.nih.gov/Blast.cgi) or the like. Besides BLAST, an algorithm called FASTA, or the like, may be utilized, as long as it is capable of calculating an appropriate identity.

As used herein, "substitution (of an amino acid)" preferably refers to a substitution within a group of conservative amino acids, in which the amino acids are similar in properties such as the electric charge, side chain, polarity, and aromaticity among the 20 kinds of amino acids constituting natural proteins. The substitution is, for example, within the uncharged polarity amino acid group having a low-polarity side chain (Gly, Asn, Gln, Ser, Thr, Cys, and Tyr), the branched-chain amino acid group (Leu, Val, and Ile), the neutral amino acid group (Gly, Ile, Val, Leu, Ala, Met, and Pro), the neutral amino acid group having a hydrophilic side chain (Asn, Gln, Thr, Ser, Tyr, and Cys), the acidic amino acid group (Asp and Glu), the basic amino acid group (Arg, Lys, and His), and the aromatic amino acid group (Phe, Tyr, and Trp). One substitution may exist alone, or 2 or more substitutions may exist. An amino acid substitution(s) in such a group is known to be less likely to change the properties of a polypeptide, and thus, is preferable.

### 1-3. Constitution

The bacteriophage of the present invention is a phage having bacteriolytic activity against bacteria of the genus *Salmonella,* and having the following constitution (herein referred to as a "2nd phage").

The 2nd phage has a genomic DNA comprising a gene encoding a tail tip protein consisting of a specific amino acid sequence and having an ability to recognize the target bacteria.

The present inventors have discovered 3 species of phages having bacteriolytic activity against bacteria of the genus *Salmonella,* and identified a tail tip protein (SEQ ID NO: 8) and a tail tip gene (SEQ ID NO: 9) from each of the genomic DNA sequences (SEQ ID NOs: 10 to 12, respectively) of these phages. The sequence identity among the genomic sequences of the 3 species of phages is 99%. The amino acid sequences of the tail tip proteins are as shown in SEQ ID NO: 8, and are completely identical with one another.

The tail tip protein consists of the amino acid sequence of SEQ ID NO: 8 composed of 637 amino acid residues. In the present invention, the tail tip protein consisting of the amino acid sequence of SEQ ID NO: 8 can allow extremely useful host specificity, i.e., being specific to bacteria of the genus *Salmonella* and having bacteriolytic activity widely against various bacterial species in the bacteria of the genus *Salmonella.*

### (1-1) Tail Tip Protein

The tail tip protein in the present invention consists of the amino acid sequence of any one of the following (a) to (c):
(a) the amino acid sequence of SEQ ID NO: 8;
(b) an amino acid sequence having addition, deletion, and/or substitution of one or a plurality of amino acids in the amino acid sequence of SEQ ID NO: 8; and
(c) an amino acid sequence having a sequence identity of 99% or more to the amino acid sequence of SEQ ID NO: 8.

The sequence identity specified in (c) is preferably 99.1% or more, 99.2% or more, 99.3% or more, 99.4% or more, 99.5% or more, 99.6% or more, 99.7% or more, 99.8% or more, or 99.9% or more.

It is preferable that, in the amino acid sequence specified in (b) or (c), the amino acid at the position corresponding to the 258th in SEQ ID NO: 8 of the tail tip protein is phenylalanine, and/or the amino acid at the position corresponding to the 617th in SEQ ID NO: 8 is serine. It should be noted that the position number is expressed on the basis of the starting methionine as the 1st.

### (1-2) Tail Tip Gene

The gene encoding the tail tip protein comprises the nucleotide sequence of any one of the following (d) to (f):
(d) the nucleotide sequence of SEQ ID NO: 9;
(e) a nucleotide sequence having addition, deletion, and/or substitution of one or a plurality of nucleotides in the nucleotide sequence of SEQ ID NO: 9; and
(f) a nucleotide sequence having a sequence identity of 95% or more to the nucleotide sequence of SEQ ID NO: 9.

Another example is a nucleotide sequence that hybridizes, under highly stringent conditions, with the nucleotide sequence complementary to the nucleotide sequence of SEQ ID NO: 9.

The sequence identity specified in (f) is preferably 96% or more, 97% or more, 98% or more, or 99% or more.

### (1-3) Genomic DNA

The 2nd bacteriophage has a genomic DNA comprising a gene encoding the tail tip protein.

The genomic DNA sequence comprises the nucleotide sequence of any one of the following (g) to (k):
(g) the nucleotide sequence of any one of SEQ ID NOs: 10 to 12;
(h) a nucleotide sequence having addition, deletion, and/or substitution of one or a plurality of nucleotides in the nucleotide sequence other than the nucleotide sequence of said gene within the nucleotide sequence of any one of SEQ ID NOs: 10 to 12;
(i) a nucleotide sequence having a sequence identity of 80% or more to the nucleotide sequence other than the nucleotide sequence of said gene within the nucleotide sequence of any one of SEQ ID NOs: 10 to 12;
(j) a nucleotide sequence having addition, deletion, and/or substitution of one or a plurality of nucleotides in the nucleotide sequence of any one of SEQ ID NOs: 10 to 12; and
(k) a nucleotide sequence having a sequence identity of 90% or more to the nucleotide sequence of any one of SEQ ID NOs: 10 to 12.

The sequence identity specified in (i) is preferably 81% or more, 82% or more, 83% or more, 84% or more, 85% or more, 86% or more, 87% or more, 88% or more, 89% or more, 90% or more, 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more.

In other words, the nucleotide sequence specified in (i) is a nucleotide sequence in which the nucleotide sequence other than a gene corresponding to the above-described gene has a sequence identity of 80% or more to the nucleotide sequence other than the nucleotide sequence of the above-described gene within the nucleotide sequence of any one of SEQ ID NOs: 10 to 12.

The sequence identity specified in (k) is preferably 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more.

In one embodiment, the 2nd phage is characterized by having a genomic DNA sequence comprising a specific nucleotide sequence, and exhibits bacteriolytic activity against target bacteria. The genomic DNA sequence of the 2nd phage is, for example, a genomic DNA sequence comprising: a nucleotide sequence of any one of SEQ ID NOs: 10 to 12 (113946 bp, 113936 bp, and 113949 bp respectively); a nucleotide sequence having addition, deletion, and/or substitution of one or a plurality of nucleotides in the nucleotide sequence of any one of SEQ ID NOs: 10 to 12; or a nucleotide sequence having a sequence identity of 80% or more, 81% or more, 82% or more, 83% or more, 84% or more, 85% or more, 86% or more, 87% or more, 88% or more, 89% or more, 90% or more, 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more to the nucleotide sequence of any one of SEQ ID NOs: 10 to 12.

### (1-4) Effects

The 2nd phage can exhibit bacteriolytic activity widely against various bacterial species of the genus *Salmonella,* and thus, can control bacteria of the *Salmonella* effectively. Additionally, the 2nd phage is also useful for treating or preventing food poisoning. The 2nd phage has a wide host range, and thus, can cover the diversity of target bacteria effectively. Accordingly, a phage that exhibits bacteriolytic activity widely against various bacterial species as the bacteriophage of the present invention does is extremely useful.

### 2. Composition

### 2-1. Overview

A second aspect of the present invention is a composition comprising the 2nd phage, and in particular a composition for controlling bacteria of the genus *Salmonella.* The composition of the present invention is characterized by comprising the bacteriophage according to the first aspect. For the composition of the present invention, target bacteria are, in particular, bacteria of the genus *Salmonella,* and in particular, *S.* Enteritidis, *S.* Typhimurium, *S.* Infantis, *S.* Montevideo, and *S.* Javiana.

The composition of the present invention can provide a pharmaceutical composition, additive (for example, food/drink additive, feed additive, or drinking-water additive), food, drink, feed, cleaning agent, disinfectant, bactericide, sanitizer, and the like that are safe for a human body, do not cause drug poisoning to the environment, and can control target bacteria.

### 2-2. Constitution

### (1) Essential Active Component

The composition of the present invention comprises, as an essential active component, the bacteriophages according to the first aspect. The composition of the present invention can bacteriolyze and control target bacteria by virtue of this active component.

The specific constitutions of the bacteriophage have been described in detail in the first aspect, and are thus omitted here.

The amount of the bacteriophage in the composition of the present invention depends on various conditions, such as the application of the composition, the subject of use, the method for use, the dosage form, and the species of a bacterium to be bacteriolyzed, but is preferably an amount sufficient for the bacteriophage to contact and infect the target bacteria in the subject of use. In the scope of common technical knowledge in the art, the amount of the bacteriophage in the composition of the present invention may be an amount effective for the bacteriophage in the composition of the present invention to control target bacteria. The titer of the phage in the composition of the present invention may be, for example, 1 × 10¹ to 1 × 10¹⁵ pfu/mL, 1 × 10³ to 1 × 10¹³ pfu/mL, 1 × 10⁵ to 1 × 10¹¹ pfu/mL, or 1 × 10⁷ to 1 × 10⁹ pfu/mL.

### (2) Another Active Component

The composition of the present invention may comprise, in addition to the bacteriophage according to the first aspect, one or more other active components having the same pharmacologic action as and/or different pharmacologic actions from the pharmacologic action of said bacteriophage to the extent that the component(s) does/do not affect the bacteriolytic activity of the phage.

The (an)other active component(s) is/are not limited to any kind. The (an)other active component(s) may be, for example, a phage(s) having bacteriolytic activity against the same bacteria as and/or different bacteria from target bacteria of the bacteriophage according to the first aspect. Such a phage may be, for example, a phage having bacteriolytic activity against bacteria of the genus *Salmonella.* Examples of phages having bacteriolytic activity against bacteria of the genus *Salmonella* include the following 1st and 3rd to 7th phages. The 1st and 3rd to 7th phages may each be used alone, or two or more species thereof may be used in combination.

### <1st Phage>

The 1st phage is a phage having bacteriolytic activity against bacteria of the genus *Salmonella,* and having the following constitution. The 1st phage has a genomic DNA sequence comprising a specific nucleotide sequence. The 1st phage comprises the nucleotide sequence of any one of the following (a) to (c), or has a genomic DNA sequence consisting of said nucleotide sequence.
(a) the nucleotide sequence of any one of SEQ ID Nos: 1 to 7;
(b) a nucleotide sequence having addition, deletion, and/or substitution of one or a plurality of nucleotides in the nucleotide sequence of any one of SEQ ID Nos: 1 to 7; and
(c) a nucleotide sequence having a sequence identity of 99% or more to the nucleotide sequence of any one of SEQ Id NOs: 1 to 7.

### <3rd Phage>

The 3rd phage is a phage having bacteriolytic activity against bacteria of the genus *Salmonella,* and having the following constitution. The 3rd phage has a genomic DNA comprising a specific nucleotide sequence. The 3rd phage comprises the nucleotide sequence of any one of the following (a) to (c), or has a genomic DNA consisting of said nucleotide sequence.
(a) the nucleotide sequence of SEQ ID NO: 13;
(b) a nucleotide sequence having addition, deletion, and/or substitution of one or a plurality of nucleotides in the nucleotide sequence of SEQ ID NO: 13; and
(c) a nucleotide sequence having a sequence identity of 90% or more to the nucleotide sequence of SEQ ID NO: 13.

### <4th Phage>

The 4th phage consists of a phage having bacteriolytic activity against bacteria of the genus *Salmonella,* and having the following constitution. The 4th phage comprises the nucleotide sequence of any one of the following (a) to (c), or has a genomic DNA sequence consisting of said nucleotide sequence.
(a) the nucleotide sequence of SEQ ID NO: 14;
(b) a nucleotide sequence having addition, deletion, and/or substitution of one or a plurality of nucleotides in the nucleotide sequence of SEQ ID NO: 14; and
(c) a nucleotide sequence having a sequence identity of 95% or more to the nucleotide sequence of SEQ ID NO: 14.

### <5th Phage>

The 5th phage consists of a phage having bacteriolytic activity against bacteria of the genus *Salmonella,* and having the following constitution. The 5th phage has a genomic DNA comprising a gene encoding an endonuclease consisting of a specific amino acid sequence and having endonuclease activity. The endonuclease in the 5th phage consists of the amino acid sequence of any one of the following (a) to (c):
(a) the amino acid sequence of SEQ ID NO: 15;
(b) an amino acid sequence having addition, deletion, and/or substitution of one or a plurality of amino acids in the amino acid sequence of SEQ ID NO: 15; and
(c) an amino acid sequence having a sequence identity of 90% or more to the amino acid sequence of SEQ ID NO: 15.

The gene encoding the endonuclease comprises, for example, the nucleotide sequence of any one of the following (d) to (f):
(d) the nucleotide sequence of SEQ ID NO: 16;
(e) a nucleotide sequence having addition, deletion, and/or substitution of one or a plurality of nucleotides in the nucleotide sequence of SEQ ID NO: 16; and
(f) a nucleotide sequence having a sequence identity of 90% or more to the nucleotide sequence of SEQ ID NO: 16.

Another example is a nucleotide sequence that hybridizes, under highly stringent conditions, with the nucleotide sequence complementary to the nucleotide sequence of SEQ ID NO: 16.

The 5th phage has a genomic DNA comprising a gene encoding an endonuclease. The genomic DNA sequence comprises, or consists of, for example, the nucleotide sequence of any one of the following (g) to (k):
(g) the nucleotide sequence of SEQ ID NO: 17;
(h) a nucleotide sequence having addition, deletion, and/or substitution of one or a plurality of nucleotides in the nucleotide sequence other than the nucleotide sequence of said gene within the nucleotide sequence of SEQ ID NO: 17;
(i) a nucleotide sequence having a sequence identity of 80% or more to the nucleotide sequence other than the nucleotide sequence of said gene within the nucleotide sequence of SEQ ID NO: 17;
(j) a nucleotide sequence having addition, deletion, and/or substitution of one or a plurality of nucleotides in the nucleotide sequence of SEQ ID NO: 17; and
(k) a nucleotide sequence having a sequence identity of 90% or more to the nucleotide sequence of SEQ ID NO: 17.

In other words, the nucleotide sequence specified in (i) is a nucleotide sequence in which the nucleotide sequence other than the gene corresponding to above-described gene has a sequence identity of 80% or more to the nucleotide sequence other than the nucleotide sequence of above-described gene in the nucleotide sequence of SEQ ID NO: 17.

In one embodiment, the 5th phage is characterized by having a genomic DNA sequence comprising a specific nucleotide sequence and exhibits bacteriolytic activity against target bacteria. The genomic DNA sequence of the 5th phage is, for example, a genomic DNA sequence comprising: a nucleotide sequence of SEQ ID NO: 17 (47638 bp); a nucleotide sequence having addition, deletion, and/or substitution of one or a plurality of nucleotides in the nucleotide sequence of SEQ ID NO: 17; or a nucleotide sequence having a sequence identity of 90% or more to the nucleotide sequence of SEQ ID NO: 17.

### <6th Phage>

The 6th phage is a phage having bacteriolytic activity against bacteria of the genus *Salmonella,* and having the following constitution. The 6th phage has a genomic DNA comprising a gene encoding a tail fiber protein consisting of a specific amino acid sequence and having an ability to recognize the target bacteria. The tail fiber protein in the 6th phage consists of the amino acid sequence of any one of the following (a) to (c):
(a) the amino acid sequence of SEQ ID NO: 18;
(b) an amino acid sequence having addition, deletion, and/or substitution of one or a plurality of amino acids in the amino acid sequence of SEQ ID NO: 18; and
(c) an amino acid sequence having a sequence identity of 99% or more to the amino acid sequence of SEQ ID NO: 18.

It is preferable that, in the amino acid sequence specified in (b) or (c), the amino acid corresponding to the 211th is Val, the amino acid corresponding to the 321st is Val, the amino acid corresponding to the 485th is Val, the amino acid corresponding to the 533rd is Ala, the amino acid corresponding to the 577th is Ser, and/or the amino acid corresponding to the 583rd is Ser. It should be noted that the position number is expressed on the basis of the starting methionine as the 1st.

The gene encoding the tail fiber protein comprises, for example, the nucleotide sequence of any one of the following (d) to (f):
(d) the nucleotide sequence of SEQ ID NO: 19;
(e) a nucleotide sequence having addition, deletion, and/or substitution of one or a plurality of nucleotides in the nucleotide sequence of SEQ ID NO: 19; and
(f) a nucleotide sequence having a sequence identity of 97% or more to the nucleotide sequence of SEQ ID NO: 19.

Another example is a nucleotide sequence that hybridizes, under highly stringent conditions, with the nucleotide sequence complementary to the nucleotide sequence of SEQ ID NO: 19.

The 6th phage has a genomic DNA comprising a gene encoding the tail fiber protein. The genomic DNA sequence comprises, for example, the nucleotide sequence of any one of the following (g) to (k):
(g) the nucleotide sequence of SEQ ID NO: 20;
(h) a nucleotide sequence having addition, deletion, and/or substitution of one or a plurality of nucleotides in the nucleotide sequence other than the nucleotide sequence of said gene within the nucleotide sequence of SEQ ID NO: 20;
(i) a nucleotide sequence having a sequence identity of 90% or more to the nucleotide sequence other than the nucleotide sequence of said gene within the nucleotide sequence of SEQ ID NO: 20;
(j) a nucleotide sequence having addition, deletion, and/or substitution of one or a plurality of nucleotides in the nucleotide sequence of SEQ ID NO: 20; and
(k) a nucleotide sequence having a sequence identity of 95% or more to the nucleotide sequence of SEQ ID NO: 20.

In other words, the nucleotide sequence specified in (i) is a nucleotide sequence in which the nucleotide sequence other than the gene corresponding to above-described gene has a sequence identity of 90% or more to the nucleotide sequence other than the nucleotide sequence of above-described gene in the nucleotide sequence of SEQ ID NO: 20.

In one embodiment, the 6th phage is characterized by having a genomic DNA sequence comprising a specific nucleotide sequence and exhibits bacteriolytic activity against target bacteria. The genomic DNA sequence of the 6th phage is, for example, a genomic DNA sequence comprising: a nucleotide sequence of SEQ ID NO: 20 (40784 bp); a nucleotide sequence having addition, deletion, and/or substitution of one or a plurality of nucleotides in the nucleotide sequence of SEQ ID NO: 20; or a nucleotide sequence having a sequence identity of 90% or more to the nucleotide sequence of SEQ ID NO: 20.

### <7th Phage>

The 7th phage consists of a phage having bacteriolytic activity against bacteria of the genus *Salmonella,* and having the following constitution. The 7th phage has a genomic DNA comprising a gene encoding a tail spike protein consisting of a specific amino acid sequence and having an ability to recognize the target bacteria. The tail spike protein in the present invention consists of the amino acid sequence of SEQ ID NO: 21. The gene encoding the tail spike protein comprises, for example, the nucleotide sequence of SEQ ID NO:22.

The 7th bacteriophage has a genomic DNA comprising a gene encoding the tail spike protein. The genomic DNA sequence comprises, or consists of, for example, the nucleotide sequence of any one of the following (a) to (e):
(a) the nucleotide sequence of SEQ ID NO: 23;
(b) a nucleotide sequence having addition, deletion, and/or substitution of one or a plurality of nucleotides in the nucleotide sequence other than the nucleotide sequence of said gene within the nucleotide sequence of SEQ ID NO: 23;
(c) a nucleotide sequence having a sequence identity of 99% or more to the nucleotide sequence other than the nucleotide sequence of said gene within the nucleotide sequence of SEQ ID NO: 23;
(d) a nucleotide sequence having addition, deletion, and/or substitution of one or a plurality of nucleotides in the nucleotide sequence of SEQ ID NO: 23; and
(e) a nucleotide sequence having a sequence identity of 99% or more to the nucleotide sequence of SEQ ID NO: 23.

In other words, the nucleotide sequence specified in (c) is a nucleotide sequence in which the nucleotide sequence other than the gene corresponding to above-described gene has a sequence identity of 99% or more to the nucleotide sequence other than the nucleotide sequence of above-described gene in the nucleotide sequence of SEQ ID NO: 23.

In one embodiment, the 7th phage is characterized by having a genomic DNA sequence comprising a specific nucleotide sequence and exhibits bacteriolytic activity against target bacteria. The genomic DNA sequence of the 7th phage is, for example, a genomic DNA sequence comprising: the nucleotide sequence of SEQ ID NO: 23 (39162 bp); a nucleotide sequence having addition, deletion, and/or substitution of one or a plurality of nucleotides in the nucleotide sequence of SEQ ID NO: 23; or a nucleotide sequence having a sequence identity of 99.0% or more to the nucleotide sequence of SEQ ID NO: 23.

The sequence identity herein is not particularly limited. Specifically, the sequence identity may be, for example, 80% or more, 81% or more, 82% or more, 83% or more, 84% or more, 85% or more, 86% or more, 87% or more, 88% or more, 89% or more, 90.0% or more, 90.5% or more, 91.0% or more, 91.5% or more, 92.0% or more, 92.5% or more, 93.0% or more, 93.5% or more, 94.0% or more, 94.5% or more, 95.0% or more, 95.5% or more, 96.0% or more, 96.5% or more, 97.0% or more, 97.5% or more, 98.0% or more, 98.5% or more, 99.0% or more, 99.1% or more, 99.2% or more, 99.3% or more, 99.4% or more, 99.5% or more, 99.6% or more, 99.7% or more, 99.8% or more, or 99.9% or more to a reference sequence.

The composition of the present invention may comprise, as an active component in combination with the bacteriophage according to the first aspect, for example, at least one phage selected from the group consisting of the above-described 1st and 3rd to 7th phages.

For example, a phage that is different from the bacteriophage according to the first aspect in terms of target bacteria or a phage that is the same as the bacteriophage in terms of target bacteria, but recognizes a different cell surface receptor can be combined with the bacteriophage according to the first aspect, and thus be expected to allow a synergistic effect and/or supportive effect of the bacteriolytic activity.

Besides, known antibiotics and the like may be included as the other active components.

### (3) Non-active Component

The composition of the present invention may further comprise a non-active component, for example, a carrier (solid carrier, liquid carrier, or the like), excipient, surfactant, emulsifying agent, binder, disintegrator, lubricant, dissolution aid, suspending agent, coating agent, colorant, flavoring agent, preservative, stabilizer, isotonizing agent, chelator, viscolizer, thickener, buffering agent, pH adjustor, and/or the like to the extent that the non-active component does not affect the bacteriolytic activity of the bacteriophage according to the first aspect.

### 2-3. Subject of Application

Examples of subjects of application for the composition of the present invention (herein often abbreviated simply as a "subject") include, but are not limited to: breeding grounds for domestic animals, such as chicken farms, pig farms, stock farms, and dairy farms (including, for example, houses, cages, and soils); food, drink, or feed; food/drink processing plants or feed manufacturing plants; processing equipment for food, drink, or feed; containers for food, drink, or feed; and any vertebrate, including a human, domestic animal (horse, bovine, sheep, goat, pig, fowl, and the like), pet animal (dog, cat, rabbit, bird, and the like), and laboratory animal (mouse, rat, monkey, and the like).

### 2-4. Form

The composition of the present invention may be in the form of a pharmaceutical composition, additive (for example, food/drink additive, feed additive, or drinking-water additive), food, drink, feed, cleaning agent, disinfectant, bactericide, sanitizer, or the like. Each form will be described below.

### (1) Pharmaceutical Composition

The composition of the present invention may be a pharmaceutical composition.

The pharmaceutical composition in the present invention can be used, for example, to control target bacteria in a subject. The pharmaceutical composition in the present invention can also be used, for example, to treat or prevent an infection caused by target bacteria. For the pharmaceutical composition in the present invention, target bacteria are, in particular, bacteria of the genus *Salmonella,* and in particular, *S.* Enteritidis, *S.* Typhimurium, *S.* Infantis, *S.* Montevideo, and *S.* Javiana.

Herein, "infection caused by bacteria of the genus *Salmonella*" is a disease induced by bacteria of the genus *Salmonella,* and is also referred to as a *Salmonella* infection or salmonellosis. A symptom of an infection caused by bacteria of the genus *Salmonella* is, for example, fever, stomachache, diarrhea, vomiturition, nausea, vomiting, or bacteremia. An infection caused by bacteria of the genus *Salmonella* may be, for example, food poisoning.

The pharmaceutical composition in the present invention may further comprise, in addition to the bacteriophage according to the first aspect, pharmaceutically acceptable the above-described non-active component (i.e., a pharmaceutical additive).

The pharmaceutical composition in the present invention may be formulated in any dosage form, for example: a solid formulation, such as a tablet, granule, powder, pill, or capsule; a liquid formulation, such as a liquid agent, suspending agent, or syrup; gel; or aerosol. It should be noted that, when used in the form of a liquid formulation, the pharmaceutical composition can also be formulated, for example, in the form of a dried product intended to be reconstituted with saline immediately before use. Additionally, in the pharmaceutical composition in the present invention, the amount of blending of the bacteriophage according to the first aspect can be set suitably. The amount of blending can be changed in accordance with the dosage form, the severity of a disease of a subject, and the like.

A subject of administration of the pharmaceutical composition in the present invention is, for example, any vertebrate including a human, domestic animal (horse, bovine, sheep, goat, pig, fowl, and the like), pet animal (dog, cat, rabbit, bird, and the like), and a laboratory animal (mouse, rat, monkey, and the like), but is preferably a human.

The route of administration of the pharmaceutical composition in the present invention is, but not limited to, oral, intravenous, rectal, transvaginal, or topical administration.

The amount of administration of the pharmaceutical composition in the present invention can be set suitably, considering various factors, such as the route of administration, the age, the body weight, and the symptom of a test subject. The pharmaceutical composition in the present invention may be administered in a single dose, or may be administered in multiple doses at intervals of several hours to several months.

### (2) Food/drink additive

The composition of the present invention may be a food/drink additive.

The food/drink additive in the present invention can be used, for example, to control target bacteria in food and/or drink. The food/drink additive in the present invention can also be added to food and/or drink to be used to thereby give a specific action (a controlling action on target bacteria or a treating or preventing action on an infection caused by target bacteria) on the food and/or drink. For the food/drink additive in the present invention, target bacteria are, in particular, bacteria of the genus *Salmonella,* and in particular, *S.* Enteritidis, *S.* Typhimurium, *S.* Infantis, *S.* Montevideo, and *S.* Javiana.

The food/drink additive in the present invention may further comprise, in addition to the bacteriophage according to the first aspect, the above-described non-active component acceptable for manufacturing food and/or drink.

The food/drink additive in the present invention may be in the form of liquid, gel, or dry powder. The kind of the food and/or drink to which the food/drink additive in the present invention is to be added is as described in "(4) Food and/or Drink".

The food/drink additive in the present invention can be added to, painted to, or sprayed on food and/or drink, using any suitable method utilizable to those skilled in the art. For example, the food/drink additive in the present invention may be mixed in a raw material for food and/or drink during production of the food and/or drink.

### (3) Feed Additive or Drinking-water Additive

The composition of the present invention may be a feed additive or a drinking-water additive. The feed additive or a drinking-water additive in the present invention may be used, for example, when a domestic animal or the like is raised.

The feed additive or a drinking-water additive in the present invention can be used, for example, to control target bacteria in a feed or drinking water. The feed additive or a drinking-water additive in the present invention can also be added to a feed or drinking water to be used to thereby give a specific action (a controlling action on target bacteria or a treating or preventing action on an infection caused by target bacteria) on the feed or drinking water. For the feed additive or a drinking-water additive in the present invention, target bacteria are, in particular, bacteria of the genus *Salmonella,* and in particular, *S.* Enteritidis, *S.* Typhimurium, *S.* Infantis, *S.* Montevideo, and *S.* Javiana.

The feed additive in the present invention may further comprise, in addition to the bacteriophage according to the first aspect, the above-described non-active component acceptable for manufacturing feed.

The feed additive in the present invention may be in the form of liquid, gel, or dry powder. The kind of feed to which the feed additive in the present invention is to be added is as described in "(5) Feed".

The feed additive in the present invention can be added to, painted to, or sprayed on feed, using any suitable method utilizable to those skilled in the art. For example, the feed additive in the present invention may be mixed in a raw material for a feed during production of the feed.

The drinking-water additive in the present invention may be in the form of liquid, gel, or dry powder. Drinking water to which the drinking-water additive in the present invention is to be added may be, for example, but is not particularly limited to, tap water, well water, groundwater, or rainwater. Drinking water may comprise another component (for example, antibiotics or the like).

The drinking-water additive in the present invention can be added to drinking water, using any suitable method utilizable to those skilled in the art. For example, the drinking-water additive in the present invention may be mixed with drinking water in a suitable container, or may be mixed with drinking water in water-supply equipment.

### (4) Food and/or Drink

The composition of the present invention may be food and/or drink.

Food and/or Drink in the present invention can be used, for example, to control target bacteria in a subject. Food and/or Drink in the present invention can also be used, for example, to treat or prevent an infection caused by target bacteria. For food and/or drink in the present invention, target bacteria are, in particular, bacteria of the genus *Salmonella,* and in particular, *S.* Enteritidis, *S.* Typhimurium, *S.* Infantis, *S.* Montevideo, and *S.* Javiana. The infection caused by target bacteria may be, for example, food poisoning.

Food and/or drink in the present invention may further comprise, in addition to the bacteriophage according to the first aspect, the above-described non-active component acceptable for manufacturing the food and/or drink.

Food and/or drink in the present invention may be in any form, for example, fresh food (vegetable, fruit, meat, seafood, cereal, or the like), processed food, precooked food, confectionery, seasoning, drink, or functional food. Examples of functional foods include; functional health foods including foods for specified health use (including conditional *tokuho* [foods for specified health use]), foods with function claims, and functional nutritional foods; special purpose foods; nutritional supplements; dietary supplements; supplements (for example, in various dosage forms, such as a tablet, coated tablet, sugar-coated tablet, capsule, and liquid agent); and foods for beauty treatment (for example, diet foods). The food and/or the drink may also be prepared in any form, for example, a solid, liquid, mixture, suspension, paste, gel, powder, granule, or capsule. Food and/or drink in the present invention may be made to comprise the bacteriophage according to the first aspect, using any suitable method utilizable to those skilled in the art. Specifically, food and/or drink in the present invention may have the bacteriophage enclosed in a capsule, may have the bacteriophage wrapped with an edible film, an edible coating agent, or the like, or may have the bacteriophage blended (supplemented) with a suitable excipient or the like, and then molded in any form, for example, a tablet. Food and/or drink in the present invention may be produced by processing a composition comprising the bacteriophage in the present invention and another food raw material. Additionally, food and/or drink in the present invention can also be produced by blending (supplementing) the bacteriophage, for example, with any of various foods (drinks, fluid diets, foods for invalids, nutritional foods, frozen foods, processed foods, other commercially available foods, and the like).

### (5) Feed

The composition of the present invention may be a feed.

The feed in the present invention can be used, for example, to control target bacteria in a subject. The feed in the present invention can also be used, for example, to treat or prevent an infection caused by target bacteria. For the feed in the present invention, target bacteria are, in particular, bacteria of the genus *Salmonella,* and in particular, *S.* Enteritidis, *S.* Typhimurium, *S.* Infantis, *S.* Montevideo, and *S.* Javiana. The infection caused by target bacteria may be, for example, food poisoning.

The feed in the present invention may further comprise, in addition to the bacteriophage according to the first aspect, the above-described non-active component acceptable for manufacturing feed.

The feed in the present invention is, for example, but not limited to, grass, straw, *Miscanthus*, hay, silage, cereal (corn, barley, wheat, rice, or the like), mixed feed, or a food processing by-product (bean curd lees, beer cake, bread crumbs, or the like). The feed may also be prepared in any form, for example, a solid, liquid, mixture, suspension, paste, gel, powder, granule, or capsule.

The feed in the present invention may be made to comprise the bacteriophage according to the first aspect, using any suitable method utilizable to those skilled in the art. Specifically, the feed in the present invention may have the bacteriophage enclosed in a capsule, may have the bacteriophage wrapped with an edible film, an edible coating agent, or the like, or may have the bacteriophage blended (supplemented) with a suitable excipient or the like, and then molded in any form, for example, a tablet. The feed in the present invention may be produced by processing a composition comprising the bacteriophage in the present invention and another feed raw material. Additionally, the feed in the present invention can also be produced by blending (supplementing) the bacteriophage, for example, with any of various feeds.

### (6) Cleaning Agent, Disinfectant, Bactericide, or Sanitizer

The composition of the present invention may be a cleaning agent, a disinfectant, a bactericide, or a sanitizer. As used herein, "cleaning agent" refers to a composition aimed at removing dirt from a subject of application. As used herein, "disinfectant" means a composition aimed at decreasing a microbial pathogen to a harmless degree in a subject of application. As used herein, "bactericide" refers to a composition aimed at killing bacteria in a subject of application. As used herein, "sanitizer" means a composition aimed at decreasing bacteria in a subject of application.

The cleaning agent, disinfectant, bactericide, or sanitizer in the present invention can be used, for example, to control target bacteria in a subject of application. For the cleaning agent, disinfectant, bactericide, or sanitizer in the present invention, target bacteria are, in particular, bacteria of the genus *Salmonella,* and in particular, *S.* Enteritidis, *S.* Typhimurium, *S.* Infantis, *S.* Montevideo, and *S.* Javiana.

The cleaning agent, disinfectant, bactericide, or sanitizer in the present invention may be in the form of liquid, gel, or dry powder.

Examples of subjects of application to which the cleaning agent, disinfectant, bactericide, or sanitizer in the present invention is to be applied include, but are not limited to: breeding grounds for domestic animals, such as chicken farms, pig farms, stock farms, and dairy farms (including, for example, houses, cages, and soils); food, drink, or feed; food/drink processing plants or feed manufacturing plants; processing equipment for food, drink, or feed; containers for food, drink, or feed; and any vertebrate, including a human, domestic animal (horse, bovine, sheep, goat, pig, fowl, and the like), pet animal (dog, cat, rabbit, bird, and the like), and laboratory animal (mouse, rat, monkey, and the like).

The cleaning agent, disinfectant, bactericide, or sanitizer in the present invention can be used in the form, for example, so as to be added to, painted to, sprayed on, or dispersed on a subject of application. The cleaning agent, disinfectant, bactericide, or sanitizer in the present invention can also be used in the form, for example, so as to have a subject of application immersed therein.

### 3. Method for Controlling Target Bacteria

### 3-1. Overview

A third aspect of the present invention is a method for controlling target bacteria. The method for controlling target bacteria according to the present invention is characterized by using the bacteriophage according to the first aspect or the composition according to the second aspect to control target bacteria. In the method for controlling target bacteria according to the present invention, target bacteria are, in particular, bacteria of the genus *Salmonella,* and in particular, *S.* Enteritidis, *S.* Typhimurium, *S.* Infantis, *S.* Montevideo, and *S.* Javiana.

The method for control according to the present invention can control target bacteria in a subject of application.

### 3-2. Method

The method for controlling target bacteria according to the present invention comprises a contacting step as an essential step.

The "contacting step" is a step of contacting the bacteriophage according to the first aspect or the composition according to the second aspect to a subject of application.

The "contact" in the present aspect refers to direct contact between the bacteriophage according to the first aspect or the composition according to the second aspect and a subject of application. More specifically, the contact means that bacteriophage according to the first aspect or the bacteriophage according to the first aspect in the composition according to the second aspect, i.e., the phage comes into contact with a subject of application, preferably a site thereof which can be at a risk of being contaminated with target bacteria. This step is intended to infect a phage as an active component with target bacteria, whereby the target bacteria are bacteriolyzed. As a result, an effect on controlling target bacteria can be achieved.

A subject of application in the method for controlling target bacteria according to the present invention is as described in the second aspect.

In the method for controlling target bacteria according to the present invention, the contacting step can be performed, for example, by adding, painting, spraying, or dispersing the bacteriophage according to the first aspect or the composition according to the second aspect (in particular, a pharmaceutical composition, food/drink additive, feed additive, drinking-water additive, cleaning agent, disinfectant, bactericide, or sanitizer) to or on a subject of application, or by immersing a subject of application in the bacteriophage according to the first aspect or the composition according to the second aspect (in particular, a pharmaceutical composition, food/drink additive, feed additive, drinking-water additive, cleaning agent, disinfectant, bactericide, or sanitizer).

The contacting step can also be performed by administering the composition according to the second aspect (in particular, a pharmaceutical composition, food, drink, or feed) to a subject of application.

### 4. Method for Treating or Preventing Infection caused by Target Bacteria

### 4-1. Overview

A fourth aspect of the present invention is a method for treating or preventing an infection caused by bacteria of the genus *Salmonella* as target bacteria. The method for treatment or prevention according to the present invention is characterized by using the bacteriophage according to the first aspect or the composition according to the second aspect to treat or prevent the infection caused by target bacteria.

### 4-2. Method

The method for treatment or prevention according to the present invention comprises an administering step as an essential step. The "administering step" is a step of administering the bacteriophage according to the first aspect or the composition according to the second aspect to a subject. In the method for treatment or prevention according to the present invention, target bacteria are, in particular, bacteria of the genus *Salmonella,* and in particular, *S.* Enteritidis, *S.* Typhimurium, *S.* Infantis, *S.* Montevideo, and *S.* Javiana.

In the method for treatment or prevention according to the present invention, a subject of administration and a method for administration (the amount of administration, the route of administration, and the frequency of administration) are as described in "2-4. (1) Pharmaceutical Composition" above.

### 5. Method for Identifying Bacteria of the Salmonella Genus

### 5-1. Overview

A fifth aspect of the present invention is a method for identifying bacteria of the genus *Salmonella,* and in particular, *S.* Enteritidis, *S.* Typhimurium, *S.* Infantis, *S.* Montevideo, and *S.* Javiana. The method for identification according to the present invention is characterized by utilizing the bacteriolytic activity of the bacteriophage according to the first aspect against bacteria of the genus *Salmonella* to identify bacteria of the genus *Salmonella* (in particular, *S.* Enteritidis, *S.* Typhimurium, *S.* Infantis, *S.* Montevideo, and *S.* Javiana).

The present invention can determine and identify whether unidentified bacteria are bacteria of the genus *Salmonella.*

### 5-2. Method

The method for identification according to the present invention comprises a culturing step, a mixing step, a mixture culturing step, and a determining step as essential steps, and additionally, an isolating step as an optional step. Each step is described below.

### (1) Isolating Step

The "isolating step" is a step of isolating a subject bacterium from a specimen suspected of comprising the bacteria of the genus *Salmonella.* This step is an optional step, and may be performed if desired.

The "subject bacterium" refers to a bacterium to be subjected to the method for identification according to the present invention, the species of which bacterium has not been revealed.

The specimen may be feces, food, drink, or feed, or may be a swab taken from a breeding ground for a domestic animal, a food/drink processing plant, a feed manufacturing plant, or the like.

When the amount of bacteria of the genus *Salmonella* in the specimen is expected to be large (for example, when the specimen is feces of a subject exhibiting salmonellosis), the specimen can be streaked directly on a agar medium, and undergo isolation culture. After the isolation culture, a single colony can be picked up to isolate the subject bacterium. When the amount of bacteria of the genus *Salmonella* in the specimen is expected to be small (for example, when the specimen is food or a swab), the specimen can be placed in a culture medium, and undergoes enrichment culture, and then, the resulting culture solution is streaked on a agar medium, and undergo isolation culture. After the isolation culture, the subject bacterium can be isolated in the similar manner as described above. When the bacteria of the genus *Salmonella* are expected to be damaged or dormant (for example, when the specimen is a processed food), the enrichment culture may be preceded by additional preliminary enrichment culture.

### (2) Culturing Step

The "culturing step" is a step of culturing the subject bacterium isolated, and obtaining a culture preparation. The subject bacterium may be cultured by a method known in the art.

The "culture preparation" is obtained by culturing the subject bacterium, and may be either liquid or solid.

In this step, the subject bacterium is unidentified, and thus, the culture medium to be used in this step is desirably a culture medium that can culture a wide range of bacteria. At least a culture medium that can culture bacteria of the genus *Salmonella,* bacteria to be identified in the present invention, is used. Such a culture medium may be, for example, a culture medium comprising one or more components selected from: protein enzymatic degradation products such as peptone and tryptone; organism-derived extracts such as potato dextrose and yeast extracts; amino acids or salts thereof, such as glutamic acid; saccharides such as glucoses, sucroses, and lactoses; and inorganic salts such as sodium chloride, magnesium chloride, potassium dihydrogen phosphate, and sodium thiosulfate. Specifically, the culture medium and the composition are, for example, an LB culture medium (Lysogeny Broth culture medium; a standard medium comprising tryptone, yeast extract, and sodium chloride), a DHL culture medium (Desoxycholate Hydrogen sulfide lactose culture medium; a culture medium for bacteria of Enterobacteriaceae, comprising desoxycholate and the like), an SS culture medium (*Salmonella-Shigella* culture medium; a selective medium for bacteria of the genus *Salmonella* and/or bacteria of the genus *Shigella*, comprising meat extract, peptone, and the like), and an RV culture medium (Rappaport-Vassiliadis culture medium; an enrichment medium for bacteria of the genus *Salmonella,* comprising peptone and the like).

The subject bacterium isolated is seeded in the culture medium, and cultured under suitable culture conditions. The culture conditions are, for example, at 20 to 40°C, 20 to 30°C, 22 to 28°C, or 24 to 26°C. When a liquid culture medium is used, a culture with stirring can yield a culture preparation. The culture time is not limited, and may be any period of time, for example, the culture may be performed until the turbidity at 600 nm reaches approximately 1.0. The present step yields a culture preparation of the subject bacterium. Additionally, the culture may be a multi-step culture comprising two or more steps. For example, a culture solution obtained by a culture in a liquid culture medium can be supplemented with a soft agar containing liquid medium, poured onto a solid medium such as an agar medium, solidified, and subjected to further culture.

### (3) Mixing Step

The "mixing step" is a step of mixing the culture preparation obtained in the culturing step and the bacteriophage according to the first aspect to obtain a mixture.

The "mixture" is a mixture of a culture preparation and the bacteriophage, and may be either liquid or solid.

The mixing method is not particularly limited, as long as it is capable of mixing the culture preparation and the bacteriophage. The bacteriophage according to the first aspect may be applied in a solid state, or may be suspended in water or a liquid culture medium, and applied in a liquid state.

When the culture preparation and the bacteriophage are both liquid, the volume ratio of the culture preparation to the bacteriophage may be 1:9, 2:8, 3:7, 4:6, 5:5, 6:4, 7:3, 8:2, or 9:1. After the application, the culture preparation and the bacteriophage may be mixed sufficiently by stirring or the like. On the other hand, when a soft agar containing liquid medium is superposed as described above, the culture preparation is solid. In this case, the bacteriophage may be dropped on a solid culture preparation, like the surface of a gel, whereby both are mixed on the solid medium to obtain a mixture.

### (4) Mixture Culturing Step

The "mixture culturing step" is a step of culturing the mixture under predetermined conditions.

In the culture of the mixture, the mixture may also be supplemented with a soft agar containing liquid medium, poured onto a solid medium such as an agar medium, solidified, and subjected to further culture.

The basic procedure in this step is in accordance with the culturing step above. In this step, the culture is preferably based on, but not limited to, what is called a plaque assay method so that the bacteriolysis of the subject bacterium by a bacteriophage can be more easily verified in the below-described determining step. For example, after part of the mixture solution is mixed with a soft agar medium having the same composition, and before the soft agar medium is solidified, the resulting mixture may be poured onto an agar medium having the same composition, and spread on the whole culture medium. Then, the mixture may be cultured under the similar conditions as in the culturing step.

### (5) Determining Step

The "determining step" is a step of determining that the subject bacterium is a bacterium of the genus *Salmonella,* when the subject bacterium is bacteriolyzed after the culturing step.

Without limitation, for example, when a plaque assay method is used, whether bacteriolysis occurs may be determined according to whether a plaque has been formed. When a plaque exists on the soft agar medium, that had been spread and solidified on the agar medium, after the above-described mixture culturing step, this result indicates that the subject bacterium has been bacteriolyzed by an infection of the bacteriophage of the present invention. Accordingly, the subject bacterium in this case can be determined to be a bacterium of the genus *Salmonella.* On the other hand, when the subject bacterium proliferates on the whole agar medium, and no plaque at all exists, the subject bacterium can be determined not to be a bacterium of the genus *Salmonella.*

To render the determination more accurate, a negative control and/or a positive control may be prepared simultaneously and used to verify that no plaque is generated in the negative control, and that a plaque is observed in the positive control, wherein the negative control is mixture with a culture medium comprising no bacteriophage in the mixture culturing step, and wherein, for the positive control, a bacterium of the genus *Salmonella* pre-identified are used from the culturing step rather than a subject bacterium.

### 5-3. Effects

The method for identifying bacteria of the genus *Salmonella* according to the present invention can identify whether the cause of food poisoning, diarrhea, vomiting, or the like is bacteria of the genus *Salmonella.* Additionally, the method for identifying bacteria of the genus *Salmonella* according to the present invention can detect whether there is contamination caused by bacteria of the genus *Salmonella.*

### [Example]

The present invention will be described more specifically below with reference to Examples. However, the technical scope of the present invention is not limited to these Examples.

### [Obtainment and Culture of Bacteria of the Salmonella Genus]

The bacterial strains used in the present Examples (Examples 1 to 7) are listed in Tables in the sections of Examples. The bacterial strains of Rakuno Gakuen University listed in Tables are bacterial strains of the genus *Salmonella,* isolated from animals in Japan. Additionally, the bacterial strains listed in Tables, and obtained from the National Institute of Animal Health, the National Agriculture and Food Research Organization (NARO) are bacterial strains of the genus *Salmonella,* isolated from fowls and chicken farms in Japan.

Additionally, the strain IDs in Tables are identification numbers allocated herein. The serotype of each bacterial strain in Tables was identified on the basis of the Kaufmann-White table of antigen structures, from the result of an agglutination test using an immune serum for *Salmonella* diagnosis (Denka Seiken Co., Ltd.). The serotype was verified also by a gene analysis technique, such as PFGE (pulsed-field gel electrophoresis) or PCR (polymerase chain reaction), if desired.

As to the bacterial strains ST1 to ST6 in Tables, the drug sensitivity, PFGE type, and the like of the bacterial strains have been examined (Yukino Tamamura, "Molecular epidemiological analysis of Salmonella enterica subsp. enterica serovar Typhimurium isolated from cattle", a thesis for doctoral degree, Department of Veterinary Medicine, The Faculty of Veterinary Medicine, Rakuno Gakuen University (2015)).

To culture the various bacteria of the genus *Salmonella,* a liquid culture medium (LB Broth) obtained by dissolving 10 g of tryptone, 5 g of yeast extract, and 10 g of sodium chloride in 1 L of H₂O, and autoclaving the resulting solution was used. Additionally, agar was added at 15 g per L to the above-described LB Broth, and the resulting mixture was autoclaved. The resulting agar medium (referred to as "LB Agar") was used as an agar medium. Furthermore, a soft agar medium (referred to as "LB Top Agar") obtained by adding agarose at 5 g per L to the above-described LB Broth, and autoclaving the resulting mixture was used as a soft agar medium that was to be superposed on the upper layer of the agar medium. The soft agar medium was stored at approximately 50°C, and utilized when necessary.

The above-described each bacterial strain in a dry powder state was suspended in 0.1 mL of LB Broth, and then subjected to a streak culture on LB Agar at 25°C, and a single colony was isolated. The isolated colony was inoculated to LB Broth, and subjected to a shaking culture at 25°C to produce a preculture solution. In main culture, the preculture solution was inoculated to LB Broth, and cultured at 25°C for 10 to 30 hours until the turbidity (Optical Density at 600 nm) reached approximately 1.0. The culture solution after the culture was directly used as bacteria suspension as it is.

### [Isolation and Purification of Phage]

A novel phage was isolated from natural dirty water or soil obtained in Japan. The method for isolating a phage was based on a conventional plaque assay method. First, dirty water from a pond, lake, or the like, or dirty water in which soil is suspended in water was filtrated through a 0.45 µm filter to prepare a phage-containing solution. Subsequently, the bacteria suspension and the phage-containing solution were mixed in equal amounts, and left at room temperature for approximately 10 minutes. Next, 0.2 mL of the bacteria/phage mixture solution was added to 3 mL of LB Top Agar, quickly mixed with a vortex mixer, and then poured on LB Agar. After the LB Top Agar was solidified, static culture was performed at 25°C for approximately 12 hours. On the bacterial lawn formed by the culture, a bacteriolytic plaque was formed. Then, a chip with its end cut was used to suck gel from the plaque portion, and a phage having bacteriolytic activity against bacteria of the genus *Salmonella* was isolated. Then, the above-described procedure was repeated to purify the phage, using a phage-containing solution comprising a high concentration of the phage isolated instead of dirty water.

The phage isolated was suspended in an SM Buffer, passed through a 0.2 µm filter, and collected as a phage-containing solution. This phage-containing solution was mixed with the bacteria suspension under the above-described conditions to isolate a phage again. This procedure was repeated several times to further purify the phage. The composition of the SM Buffer is shown in Table below.

**[Table 1]**

| SM Buffer | Final | Add to 1 L |
|---|---|---|
| NaCl | 0.1 M | 5.8 g |
| Mg₂SO₄·7H₂O | 10 mM | 1 g |
| 1M Tris-HCl pH 8.0 | 50 mM | 50 mL |
| Gelatin | 0.1 % | 0.1 g |

### [Amplification and Refinement of Phage]

The phage isolated and purified was amplified and refined by the plate lysate (PL) method that is an amplifying method using a plaque assay method. In order for many plaques to be formed on LB Agar, a bacteria/phage mixture solution was prepared, mixed with LB Top Agar, then spread on LB Agar, and cultured. Then, 3 mL of SM Buffer was added to the LB Top Agar having plaques formed thereon, and shaken at 25°C for approximately 30 minutes, and the supernatant was passed through a 0.2 µm filter to collect a collected solution containing a phage.

PEG 6000 (at a final concentration of 10%) in an amount of 1 g and 0.4 g of NaCl (at a final concentration of 4%) were added to and dissolved in 10 mL of the collected solution, and the resulting solution was rotated using a rotator at 4°C overnight. Then, the supernatant was removed by centrifugation under 15,000 × g at 4°C for 60 minutes. The pellets collected were suspended in 0.5 mL of SM Buffer again. Subsequently, 0.5 mL of chloroform was added, and the resulting mixture was stirred vigorously, and left on ice for 6 hours. After centrifugation under 8,000 × g at 4°C for 10 minutes, the upper layer was collected carefully to obtain a refined solution of phage. The concentration of the refined solution of phage is commonly expressed as a titer [PFU/mL] based on the number of plaques (Plaque Forming Unit, PFU) in accordance with a plaque assay method, and serves as one index indicative of bacteriolytic activity. The titer of the refined solution of phage prepared was determined by a plaque assay method using a solution suitably diluted.

### [Evaluation of Host Range of Phage]

The host range of the phage was evaluated by a spot test method. Only a bacteria suspension in an amount of 0.1 mL was added to and mixed with 3 mL of LB Top Agar, then poured onto LB Agar, spread on the whole plate, and solidified. As a bacteria suspension, a bacteria suspension of bacteria of the genus *Salmonella* prepared in each Example was used. Then, approximately 5 µL of the refined solution of phage was dropped onto the plate, and subjected to a static culture at 25°C for approximately 12 hours. When a clear circular shape (approximately 1 cm in diameter) was observed at the site where the phage was dropped on the plate having the bacterial lawn formed therein, it was determined that the phage dropped had bacteriolytic activity against the bacterial strain.

### [Preparation and Sequencing of Genomic DNA of Phage]

The genome of the phage was extracted using a TURBO DNA-free^{™} kit (Thermo Fisher Scientific Inc.). Host bacteria-derived genomic DNAs, which become contaminants, were removed by a treatment according to the manual attached to the kit. Then, using NucleoSpin (registered trademark) Virus (Machery-Nagel & Co. KG), the outer-shell molecules of the phage were degraded by a Proteinase K treatment according to the attached manual. Via refinement of the genomic DNA by using a silica spin column, a genomic DNA solution of the phage was prepared. Then, the concentration of the genomic DNA was measured using a Qubit dsDNA HS Assay kit (Thermo Fisher Scientific Inc.), and 50 µL of the genomic DNA solution was prepared at a final concentration of 0.2 ng/µL. Subsequently, by using Nextera XT DNA Library Prep (Illumina, Inc.) according to the attached manual, the genome of the phage was fragmented, and an adapter sequence was added by PCR. Next, Agilent High Sensitivity DNA Kit (Agilent Technologies, Inc.) was used for electrophoresis with Bioanalyzer (Agilent Technologies, Inc.) to measure the average bp size of the sample, and determine the concentration of the DNA fragments. Lastly, using a Miseq Reagent kit (Illumina, Inc.), a sample for measurement was prepared by a treatment according to the attached manual, and a measurement was made using a next-generation sequencer Miseq (Illumina, Inc.). Utilizing a CLC genomics workbench (Qiagen N.V.), the data obtained was pretreated (for example, trimmed), and then subjected to a de novo assembly to obtain the contig sequence corresponding to the genomic sequence of the phage.

### <Example 1: Isolation of 1st Bacteriophage, and Bacteriolytic Activity Thereof>

### (Purpose)

Novel bacteriophages having bacteriolytic activity against bacteria of the genus *Salmonella* are isolated, and their bacteriolytic activity against bacteria of the genus *Salmonella* are verified in this Example.

### (Method and Results)

### (1) Obtainment and Culture of Bacteria of the Salmonella Genus

The bacterial strains used in Example 1 are listed in Table below.

**[Table 2]**

| Strain ID | Serotype | Strain Name | Source of Supplv |
|---|---|---|---|
| SE6 | *S*. Entertidis | L-2728 | The National Institute of Animal Health, The National Agriculture and Food Research Organization |
| SE8 | *S*. Entertidis | L-2844 | The National Institute of Animal Health, The National Agriculture and Food Research Organization |
| SE12 | *S*. Entertidis | L-3164 | The National Institute of Animal Health, The National Agriculture and Food Research Organization |
| SE17 | *S*. Entertidis | L-3782 | The National Institute of Animal Health, The National Agriculture and Food Research Organization |
| SE18 | *S*. Entertidis | L-5104 | The National Institute of Animal Health, The National Agriculture and Food Research Organization |
| ST1 | *S*. Typhimurium | HRS-TST-129 | The Faculty of Veterinary Medicine, Rakuno Gakuen University |
| ST4 | *S*. Typhimurium | HRS-KST-31 | The Faculty of Veterinary Medicine, Rakuno Gakuen University |
| ST6 | *S*. Typhimurium | HRS-U1 | The Faculty of Veterinary Medicine, Rakuno Gakuen University |
| SI1 | *S*. Infantis | O7:Hr70A | The Faculty of Veterinary Medicine, Rakuno Gakuen University |
| SI3 | *S*. Infantis | O7:Hr1.5 | The Faculty of Veterinary Medicine, Rakuno Gakuen University |
| SM | *S*. Montevideo | S. Montevideo No.1 | The Faculty of Veterinary Medicine, Rakuno Gakuen University |
| SJ | *S*. Javiana | L-750 | The Faculty of Veterinary Medicine, Rakuno Gakuen University |

### (2) Isolation and Purification of 1st Phage

In accordance with the method described in the section of [Isolation and Purification of Phage] above, 7 species of new phages (corresponding to the 1st phages) were isolated from natural dirty water and soil, and purified.

### (3) Amplification and Refinement of 1st Phage

In accordance with the method described in the section of [Amplification and Refinement of Phage] above, a refined solution of the 1st phage was prepared, and the titer was measured. The titer was confirmed to be 10⁸ PFU/mL or more.

### (4) Evaluation of Host Range of 1st Phage

In accordance with the method described in the section of [Evaluation of Host Range of Phage] above, the host range of the 1st phage was evaluated by a spot test method.

One example of the results is shown in Figure 1 and Figure 2. The 7 species of 1st phages obtained in the present Example exhibited bacteriolytic activity against various bacterial strains of S. Enteritidis, but did not exhibit bacteriolytic activity against any of S. Typhimurium, S. Infantis, S. Montevideo, and S. Javiana.

S. Enteritidis is a serotype that is detected with the highest frequency in the food poisoning of a human (Oh and Park, J. Microbiol. Biotechnol. (2017), 27(12), 2075-2088). Accordingly, the 1st phage is particularly useful, for example, for treating or preventing food poisoning of a human. Additionally, the 1st phage exhibited bacteriolytic activity specifically against S. Enteritidis, and thus, is particularly useful for identifying S. Enteritidis.

### (5) Genomic Analysis of 1st Phage

The genomic DNA sequences of the 1st phages were determined and analyzed.

### (i) Preparation and Sequencing of Genomic DNA of 1st Phage

In accordance with the method described in the section of [Preparation and Sequencing of Genomic DNA of Phage] above, the genomic DNA sequences of the 1st phages were determined. The genomic DNA sequences determined of 7 species of 1st phages are shown in SEQ ID NOs: 1 to 7.

### (ii) Bioinformatics Analysis Based on Genomic Sequence Information

The genomic DNA sequences (SEQ ID NOs: 1 to 7) of the 1st phages were found to have a high sequence identity thereamong. The sequence identities of the shortest genomic DNA sequence SEQ ID NO: 7 to the genomic DNA sequences SEQ ID NO: 1 to 6 were calculated using the genetic information processing software GENETYX (https://www.genetyx.co.jp/), and, as a result, were consequently found to be 100% over the whole range.

Using the genomic DNA sequences of SEQ ID NOs: 2 and 7 as query sequences, similar DNA sequences were searched for on the NCBI-provided BLAST server (https://blast.ncbi.nlm.nih.gov/Blast.cgi). Prior literature on the host range was further investigated for a phage having a genomic DNA sequence having a high sequence identity to SEQ ID NO: 2 or 7 in the whole range. As a result, there was found no phage that has a genomic DNA sequence having a sequence identity estimated to be 99% or more to SEQ ID NO: 2 or 7 in the whole range, and is known to have the same host range as the 1st phage.

### <Example 2: Isolation of 2nd Bacteriophage, and Bacteriolytic Activity Thereof>

### (Purpose)

Novel bacteriophages having bacteriolytic activity against bacteria of the *Salmonella* are isolated, and their bacteriolytic activity against bacteria of the genus *Salmonella* are verified in this Example.

### (Method and Results)

### (1) Obtainment and Culture of Bacteria of the Salmonella Genus

The bacterial strains used in Example 2 are listed in Table below.

**[Table 3]**

| Strain ID | Serotype | Strain Name | Source of Supply |
|---|---|---|---|
| SE1 | *S*. Entertidis | L-2596 | The National Institute of Animal Health, The National Agriculture and Food Research Organization |
| SE2 | *S*. Entertidis | L-2653 | The National Institute of Animal Health, The National Agriculture and Food Research Organization |
| SE3 | *S*. Entertidis | L-2602 | The National Institute of Animal Health, The National Agriculture and Food Research Organization |
| SE4 | *S*. Entertidis | L-2685 | The National Institute of Animal Health, The National Agriculture and Food Research Organization |
| SE5 | *S*. Entertidis | L-2712 | The National Institute of Animal Health, The National Agriculture and Food Research Organization |
| SE6 | *S*. Entertidis | L-2728 | The National Institute of Animal Health, The National Agriculture and Food Research Organization |
| SE7 | *S*. Entertidis | L-2777 | The National Institute of Animal Health, The National Agriculture and Food Research Organization |
| SE8 | *S*. Entertidis | L-2844 | The National Institute of Animal Health, The National Agriculture and Food Research Organization |
| SE9 | *S*. Entertidis | L-2916 | The National Institute of Animal Health, The National Agriculture and Food Research Organization |
| SE10 | *S*. Entertidis | L-2917 | The National Institute of Animal Health, The National Agriculture and Food Research Organization |
| SE11 | *S*. Entertidis | L-3080 | The National Institute of Animal Health, The National Agriculture and Food Research Organization |
| SE12 | *S*. Entertidis | L-3164 | The National Institute of Animal Health, The National Agriculture and Food Research Organization |
| SE13 | *S*. Entertidis | L-3241 | The National Institute of Animal Health, The National Agriculture and Food Research Organization |
| SE14 | *S*. Entertidis | L-3244 | The National Institute of Animal Health, The National Agriculture and Food Research Organization |
| SE15 | *S*. Entertidis | L-3246 | The National Institute of Animal Health, The National Agriculture and Food Research Organization |
| SE16 | *S*. Entertidis | L-3247 | The National Institute of Animal Health, The National Agriculture and Food Research Organization |
| SE17 | *S*. Entertidis | L-3782 | The National Institute of Animal Health, The National Agriculture and Food Research Organization |
| SE18 | *S*. Entertidis | L-5104 | The National Institute of Animal Health, The National Agriculture and Food Research Organization |
| ST3 | *S*. Typhimurium | HRS-TST-219 | The Faculty of Veterinary Medicine, Rakuno Gakuen University |
| SI1 | *S*. Infantis | O7:Hr70A | The Faculty of Veterinary Medicine, Rakuno Gakuen University |
| SI2 | *S*. Infantis | O7:Hd70B | The Faculty of Veterinary Medicine, Rakuno Gakuen University |
| SI3 | *S*. Infantis | O7:Hr1.5 | The Faculty of Veterinary Medicine, Rakuno Gakuen University |
| SM | *S*. Montevideo | S. Montevideo No.1 | The Faculty of Veterinary Medicine, Rakuno Gakuen University |
| SJ | *S*. Javiana | L-750 | The Faculty of Veterinary Medicine, Rakuno Gakuen University |

### (2) Isolation and Purification of 2nd Phage

In accordance with the method described in the section of [Isolation and Purification of Phage] above, 3 species of new phages (corresponding to the 2nd phage) were isolated from natural dirty water and soil, and purified.

### (3) Amplification and Refinement of 2nd Phage

In accordance with the method described in the section of [Amplification and Refinement of Phage] above, a refined solution of the 2nd phage was prepared, and the titer was measured. The titer was confirmed to be 10⁸ PFU/mL or more.

### (4) Evaluation of Host Range of 2nd Phage

In accordance with the method described in the section of [Evaluation of Host Range of Phage] above, the host range of the 2nd phage was evaluated by a spot test method.

One example of the results is shown in Figure 3 to Figure 5. The 3 species of 2nd phages obtained in the present Example exhibited bacteriolytic activity against various bacterial strains, and exhibited bacteriolytic activity against any bacterial strain of S. Enteritidis, S. Typhimurium, S. Infantis, S. Montevideo, and S. Javiana that were tested. Any of these bacterial strains is a serotype detected with a high frequency in the food poisoning of a human. Accordingly, the 2nd phage is particularly useful, for example, for treating or preventing food poisoning of a human.

### (5) Genomic Analysis of 2nd Phage

The genomic DNA sequences of the 2nd phages were determined and analyzed.

### (i) Preparation and Sequencing of Genomic DNA of 2nd Phage

In accordance with the method described in the section of [Preparation and Sequencing of Genomic DNA of Phage] above, the genomic DNA sequences of the 2nd phages were determined. The genomic DNA sequences determined of the 3 species of 2nd phages are shown in SEQ ID NOs: 10 to 12.

### (ii) Bioinformatics Analysis Based on Genomic Sequence Information

The sequence identity among the genomic DNA sequences (SEQ ID NOs: 10 to 12) of the 3 species of phages obtained is 99%. The amino acid sequences of the tail tip proteins are as shown in SEQ ID NO: 8, and are completely identical with one another.

DNA sequences similar to the genomic DNA sequences (SEQ ID NOs: 10 to 12) of the 3 species of phages obtained were searched for, and the respective sequence identities therebetween were verified, using the NCBI-provided BLAST server (https://blast.ncbi.nlm.nih.gov/Blast.cgi). As a result of the search, the nucleotide sequence having the highest identity was the genomic sequence of *Salmonella* phage S124 (GenBank Accession No.: NC_048013.1), and the sequence identity in the whole range was 79.14% (the Query Cover/Per.Ident value was 83%/95.36%).

To verify the cause for a difference in the host range between the 3 species of phages obtained and S124, the sequences of the tail tip proteins of both phages were compared. From the 3 species of phages obtained, the tail tip protein gene was identified. The gene was identified utilizing the RAST server (https://rast.nmpdr.org/) and the PHASTER server (https://phaster.ca/). Consequently, the nucleotide sequence of SEQ ID NO: 9 was identified as the gene of the tail tip protein.

Furthermore, the amino acid sequence (SEQ ID NO: 8) encoded by the gene comprising the nucleotide sequence of SEQ ID NO: 9 was compared with the amino acid sequence (the access code: YP_009806053.1) of the analogous protein of S124, with the result that the sequence identity was 97.49%. Conceivably, it is highly possible that this difference in the sequence is in connection with a difference in the host range. When using, as a query sequence, only the amino acid sequence (SEQ ID NO: 8) of the tail tip protein of the 3 species of phages obtained, the NCBI-provided BLAST server was searched, and 4 known sequences having a sequence identity of 95% or more were detected. The alignment between the query sequence and the sequences searched for is shown in Figure 11. In Figure 11, the amino acid sequence of "HCH9411546.1" is shown in SEQ ID NO: 24, the amino acid sequence of "YP_009966103.1" is shown in SEQ ID NO: 25, the amino acid sequence of "YP_009194791.1" is shown in SEQ ID NO: 26, and the amino acid sequence of "YP_009806053.1" is shown in SEQ ID NO: 27. Among these 4 known sequences, the sequences other than S124 have been reported with no detailed information on the host range, or are sequences derived from a prophage. It is understood that F (Phe) at the 258th and S (Ser) at the 617th in the query sequence (the amino acid sequence of the tail tip protein of the 3 species of phages obtained) are unique amino acid residues found in only the query sequence, unlike the corresponding residues of the 4 known sequences. Presumably, the characteristics in these sequences are in connection with the bacteriolytic activity of the 2nd phage against a wide range of serotypes.

### <Example 3: Isolation of 3rd Bacteriophage, and Bacteriolytic Activity Thereof>

### (Purpose)

A novel bacteriophage having bacteriolytic activity against bacteria of the genus *Salmonella* is isolated, and its bacteriolytic activity against bacteria of the genus *Salmonella* is verified in this Example.

### (Method and Results)

### (1) Obtainment and Culture of Bacteria of the Salmonella Genus

The bacterial strains used in Example 3 are listed in Table below.

**[Table 4]**

| Strain ID | Serotype | Strain Name | Source of Supplv |
|---|---|---|---|
| ST1 | *S.* Typhimurium | HRS-TST-129 | The Faculty of Veterinary Medicine, Rakuno Gakuen University |
| ST2 | *S.* Typhimurium | HRS-TST-139 | The Faculty of Veterinary Medicine, Rakuno Gakuen University |
| ST3 | *S.* Typhimurium | HRS-TST-219 | The Faculty of Veterinary Medicine, Rakuno Gakuen University |
| ST4 | *S.* Typhimurium | HRS-KST-31 | The Faculty of Veterinary Medicine, Rakuno Gakuen University |

### (2) Isolation and Purification of 3rd Phage

In accordance with the method described in the section of [Isolation and Purification of Phage] above, 1 species of new phage (corresponding to the 3rd phage) was isolated from natural dirty water and soil, and purified.

### (3) Amplification and Refinement of 3rd Phage

In accordance with the method described in the section of [Amplification and Refinement of Phage] above, a refined solution of the 3rd phage was prepared, and the titer was measured. The titer was confirmed to be 10⁸ PFU/mL or more.

### (4) Evaluation of Host Range of 3rd Phage

In accordance with the method described in the section of [Evaluation of Host Range of Phage] above, the host range of the 3rd phage was evaluated by a spot test method.

One example of the results is shown in Figure 6. The 1 species of 3rd phage obtained in the present Example exhibited bacteriolytic activity against various bacterial strains of S. Typhimurium. S. Typhimurium is a serotype detected with a high frequency in the food poisoning of a human.

Additionally, S. Typhimurium is known to be bacteria which has drug resistance and is less susceptible to antibiotics. For example, S. Typhimurium used in the present Example has been confirmed to be multidrug-resistant to various antibiotics. Specifically, it is known that ST1, ST4, ST2, and ST3 have drug resistance to S/Su, A/C/S/Su/T, A/C/Su, and A/S/Su/T respectively (Yukino Tamamura, "Molecular epidemiological analysis of *Salmonella enterica* subsp. *enterica* serovar Typhimurium isolated from cattle", a thesis for doctoral degree, Department of Veterinary Medicine, Rakuno Gakuen University (2015)). It should be noted that A represents ampicillin, C represents chloramphenicol, S represents streptomycin, Su represents sulfonamides, and T represents tetracycline. Accordingly, the 3rd phage can effectively control *S.* Typhimurium, which has drug resistance and is less susceptible to antibiotics, and the 3rd phage is particularly useful for treating or preventing food poisoning of a human.

Furthermore, although not shown in the drawings, it was verified that the 3rd phage exhibits bacteriolytic activity against ST5 (S. Typhimurium HRS-KST-203, The Faculty of Veterinary Medicine, Rakuno Gakuen University) and ST6 (S. Typhimurium HRS-U1, The Faculty of Veterinary Medicine, Rakuno Gakuen University).

### (5) Genomic Analysis of 3rd Phage

The genomic DNA sequence of the 3rd phage was determined and analyzed.

### (i) Preparation and Sequencing of Genomic DNA of 3rd Phage

In accordance with the method described in the section of [Preparation and Sequencing of Genomic DNA of Phage] above, the genomic DNA sequence of the 3rd phage was determined. The genomic DNA sequence determined of the 1 species of 3rd phage is shown in SEQ ID NO: 13.

### (ii) Bioinformatics Analysis Based on Genomic Sequence Information

Using the genomic DNA sequence (SEQ ID NO: 13) of the 3rd phage as a query sequence, a search for a similar DNA sequence and verification of the sequence identity therebetween were performed on the NCBI-provided BLAST server (https://blast.ncbi.nlm.nih.gov/Blast.cgi). As a result of the search, the nearest-neighbor nucleotide sequence was the genomic sequence of *Escherichia* phage vB_EcoM-RPN242 (GenBank Accession No.: OL656110.1), and the sequence identity in the whole range was 87.85% (the Query Cover/Per.Ident value was 89%/98.71%). Another nearest-neighbor nucleotide sequence having a sequence identity of approximately 85% was the genomic sequence of *Escherichia* phage vB_EcoM-ZQ1 (GenBank Accession No.: MW650886.1), and the sequence identity in the whole range was 84.35% (the Query Cover/Per.Ident value was 86%/98.09%). For none of the phages, the host is bacteria of the genus *Salmonella,* thus suggesting that the 3rd phage is a phage having a novel genomic sequence, the analogous genomic sequence of which is not known at all.

### <Example 4: Isolation of 4th Bacteriophage, and Bacteriolytic Activity Thereof>

### (Purpose)

A novel bacteriophage having bacteriolytic activity against bacteria of the *Salmonella* is isolated, and its bacteriolytic activity against bacteria of the genus *Salmonella* is verified in this Example.

### (Method and Results)

### (1) Obtainment and Culture of Bacteria of the Salmonella Genus

The bacterial strains used in Example 4 are listed in Table below.

**[Table 5]**

| Strain ID | Serotype | Strain Name | Source of Supplv |
|---|---|---|---|
| SE1 | *S*. Entertidis | L-2596 | The National Institute of Animal Health, The National Agriculture and Food Research Organization |
| SE2 | *S*. Entertidis | L-2653 | The National Institute of Animal Health, The National Agriculture and Food Research Organization |
| SE8 | *S*. Entertidis | L-2844 | The National Institute of Animal Health, The National Agriculture and Food Research Organization |
| SE10 | *S*. Entertidis | L-2917 | The National Institute of Animal Health, The National Agriculture and Food Research Organization |
| SE11 | *S*. Entertidis | L-3080 | The National Institute of Animal Health, The National Agriculture and Food Research Organization |
| ST2 | *S*. Typhimurium | HRS-TST-139 | The Faculty of Veterinary Medicine, Rakuno Gakuen University |
| ST3 | *S*. Typhimurium | HRS-TST-219 | The Faculty of Veterinary Medicine, Rakuno Gakuen University |
| ST6 | *S*. Typhimurium | HRS-U1 | The Faculty of Veterinary Medicine, Rakuno Gakuen University |
| SI1 | *S*. Infantis | O7:Hr70A | The Faculty of Veterinary Medicine, Rakuno Gakuen University |
| SI3 | *S*. Infantis | O7:Hr1.5 | The Faculty of Veterinary Medicine, Rakuno Gakuen University |
| SJ | *S*. Javiana | L-750 | The Faculty of Veterinary Medicine, Rakuno Gakuen University |
| SM | *S*. Montevideo | S. Montevideo No.1 | The Faculty of Veterinary Medicine, Rakuno Gakuen University |

### (2) Isolation and Purification of 4th Phage

In accordance with the method described in the section of [Isolation and Purification of Phage] above, a new phage (corresponding to the 4th phage) was isolated from natural dirty water and soil, and purified.

### (3) Amplification and Refinement of 4th Phage

In accordance with the method described in the section of [Amplification and Refinement of Phage] above, a refined solution of the 4th phage was prepared, and the titer was measured. The titer was confirmed to be 10⁸ PFU/mL or more.

### (4) Evaluation of Host Range of 4th Phage

In accordance with the method described in the section of [Evaluation of Host Range of Phage] above, the host range of the 4th phage was evaluated by a spot test method.

One example of the results is shown in Figure 7. The 4th phage obtained in the present Example exhibited bacteriolytic activity against S. Montevideo, but did not exhibit bacteriolytic activity against any of S. Enteritidis, S. Typhimurium, S. Infantis, and S. Javiana.

### (5) Genomic Analysis of 4th Phage

The genomic DNA sequence of the 4th phage was determined and analyzed.

### (i) Preparation and Sequencing of Genomic DNA of 4th Phage

In accordance with the method described in the section of [Preparation and Sequencing of Genomic DNA of Phage] above, the genomic DNA sequence of the 4th phage was determined. The genomic DNA sequence determined of the 4th phage is shown in SEQ ID NO: 14.

### (ii) Bioinformatics Analysis Based on Genomic Sequence Information

Using the genomic DNA sequence of the 4th phage as a query sequence, a similar DNA sequence was searched for on the NCBI-provided BLAST server (https://blast.ncbi.nlm.nih.gov/Blast.cgi). As a result of the search, the nearest-neighbor DNA sequence was the genomic sequence of *Escherichia coli* bacteriophage esc-cop-9 (SEQ ID NO: 1 in U.S. Patent Application Publication No. 2019/0321423), and the sequence identity in the whole range was estimated to be 90.68%. However, a phage having a sequence identity of 95% or more in the whole range and a host of which is the bacteria of the genus *Salmonella* was not found.

### <Example 5: Isolation of 5th Bacteriophage, and Bacteriolytic Activity Thereof>

### (Purpose)

A novel bacteriophage having bacteriolytic activity against bacteria of the *Salmonella* is isolated, and its bacteriolytic activity against bacteria of the genus *Salmonella* is verified in this Example.

### (Method and Results)

### (1) Obtainment and Culture of Bacteria of the Salmonella Genus

The bacterial strains used in Example 5 are listed in Table below.

**[Table 6]**

| Strain ID | Serotype | Strain Name | Source of Supplv |
|---|---|---|---|
| ST3 | *S*. Typhimurium | HRS-TST-219 | The Faculty of Veterinary Medicine, Rakuno Gakuen University |
| ST4 | *S*. Typhimurium | HRS-KST-31 | The Faculty of Veterinary Medicine, Rakuno Gakuen University |
| ST5 | *S*. Typhimurium | HRS-KST-203 | The Faculty of Veterinary Medicine, Rakuno Gakuen University |
| ST6 | *S*. Typhimurium | HRS-U1 | The Faculty of Veterinary Medicine, Rakuno Gakuen University |

### (2) Isolation and Purification of 5th Phage

In accordance with the method described in the section of [Isolation and Purification of Phage] above, a new phage (corresponding to the 5th phage) was isolated from natural dirty water and soil, and purified.

### (3) Amplification and Refinement of 5th Phage

In accordance with the method described in the section of [Amplification and Refinement of Phage] above, a refined solution of the 5th phage was prepared, and the titer was measured. The titer was confirmed to be 10⁸ PFU/mL or more.

### (4) Evaluation of Host Range of 5th Phage

In accordance with the method described in the section of [Evaluation of Host Range of Phage] above, the host range of the 5th phage was evaluated by a spot test method.

One example of the results is shown in Figure 8. The 5th phage obtained in the present Example exhibited very high bacteriolytic activity against S. Typhimurium. Additionally, using the same method, bacteriolytic activity against bacteria of the genus *Salmonella* of other serotypes comprising S. Enteritidis was examined, but the 5th phage did not exhibit bacteriolytic activity against the bacteria of the genus *Salmonella* of the other serotypes.

### (5) Genomic Analysis of 5th Phage

The genomic DNA sequence of the 5th phage was determined and analyzed.

### (i) Preparation and Sequencing of Genomic DNA of 5th Phage

In accordance with the method described in the section of [Preparation and Sequencing of Genomic DNA of Phage] above, the genomic DNA sequence of the 5th phage was determined. The genomic DNA sequence determined of the 5th phage is shown in SEQ ID NO: 17.

### (ii) Bioinformatics Analysis Based on Genomic Sequence Information

Using the genomic DNA sequence of the 5th phage as a query sequence, a similar DNA sequence was searched for on the NCBI-provided BLAST server (https://blast.ncbi.nlm.nih.gov/Blast.cgi). As a result of the search, the nearest-neighbor nucleotide sequence was the genomic sequence of *Salmonella* phage Skate (GenBank Accession No.: NC_054639.1), and the sequence identity in the whole range was estimated at 86.61%. The genomic DNA sequences of both phages were compared in detail, resulted in revealing that the region corresponding to the region from the 2385th to the 3606th in the genomic DNA sequence of the 5th phage has been deleted in Skate. In this region, the gene (the 2434th to 3000th in SEQ ID NO: 17) encoding an endonuclease exists. The amino acid sequence of said endonuclease and the nucleotide sequence encoding the amino acid sequence are shown in SEQ ID NOs: 15 and 16 respectively. It is known that a nuclease participates in the mechanism of shutdown of the replication of a host genome. Accordingly, this result suggested that the 5th phage having the endonuclease gene can efficiently shut down the replication of the host genome, hence 5th phage has high bacteriolytic activity.

Using the amino acid sequence of the endonuclease as a query sequence, a similar amino acid sequence was searched for on the BLAST server, with the result that there was no genomic sequence of a phage having a nuclease sequence having a sequence identity of 50% or more. Accordingly, the result has revealed that the 5th phage is a novel phage having a novel endonuclease gene.

### <Example 6: Isolation of 6th Bacteriophage, and Bacteriolytic Activity Thereof>

### (Purpose)

A novel bacteriophage having bacteriolytic activity against bacteria of the genus *Salmonella* is isolated, and its bacteriolytic activity against bacteria of the genus *Salmonella* is verified in this Example.

### (Method and Results)

### (1) Obtainment and Culture of Bacteria of the Salmonella Genus

The bacterial strains used in Example 6 are listed in Table below.

**[Table 7]**

| Strain ID | Serotype | Strain Name | Source of Supplv |
|---|---|---|---|
| SE1 | *S.* Entertidis | L-2596 | The National Institute of Animal Health, The National Agriculture and Food Research Organization |
| SE4 | *S.* Entertidis | L-2685 | The National Institute of Animal Health, The National Agriculture and Food Research Organization |
| SE6 | *S.* Entertidis | L-2728 | The National Institute of Animal Health, The National Agriculture and Food Research Organization |
| SE8 | *S.* Entertidis | L-2844 | The National Institute of Animal Health, The National Agriculture and Food Research Organization |
| ST1 | *S.* Typhimurium | HRS-TST-129 | The Faculty of Veterinary Medicine, Rakuno Gakuen University |
| ST4 | *S.* Typhimurium | HRS-KST-31 | The Faculty of Veterinary Medicine, Rakuno Gakuen University |
| ST5 | *S.* Typhimurium | HRS-KST-203 | The Faculty of Veterinary Medicine, Rakuno Gakuen University |
| ST6 | *S.* Typhimurium | HRS-U1 | The Faculty of Veterinary Medicine, Rakuno Gakuen University |
| SJ | *S.* Javiana | L-750 | The Faculty of Veterinary Medicine, Rakuno Gakuen University |
| SI1 | *S.* Infantis | O7:Hr70A | The Faculty of Veterinary Medicine, Rakuno Gakuen University |
| SI3 | *S.* Infantis | O7:Hr1.5 | The Faculty of Veterinary Medicine, Rakuno Gakuen University |
| SM | *S.* Montevideo | S. Montevideo No.1 | The Faculty of Veterinary Medicine, Rakuno Gakuen University |

### (2) Isolation and Purification of 6th Phage

In accordance with the method described in the section of [Isolation and Purification of Phage] above, 1 species of new phage (corresponding to the 6th phage) was isolated from natural dirty water and soil, and purified.

### (3) Amplification and Refinement of 6th Phage

In accordance with the method described in the section of [Amplification and Refinement of Phage] above, a refined solution of the 6th phage was prepared, and the titer was measured. The titer was confirmed to be 10⁸ PFU/mL or more.

### (4) Evaluation of Host Range of 6th Phage

In accordance with the method described in the section of [Evaluation of Host Range of Phage] above, the host range of the 6th phage was evaluated by a spot test method.

One example of the results is shown in Figure 9. The 1 species of 6th phage obtained in the present Example exhibited bacteriolytic activity against a plurality of bacterial strains tested, and specifically, exhibited bacteriolytic activity against bacterial strains of S. Enteritidis, S. Typhimurium, and S. Javiana. Any of these bacterial strains is a serotype detected with a high frequency in the food poisoning of a human. Accordingly, the 6th phage is particularly useful, for example, for treating or preventing food poisoning of a human.

### (5) Genomic Analysis of 6th Phage

The genomic DNA sequence of the 6th phage was determined and analyzed.

### (i) Preparation and Sequencing of Genomic DNA of 6th Phage

In accordance with the method described in the section of [Preparation and Sequencing of Genomic DNA of Phage] above, the genomic DNA sequence of the 6th phage was determined. The genomic DNA sequence determined of the 1 species of 6th phage is shown in SEQ ID NO: 20.

### (ii) Bioinformatics Analysis Based on Genomic Sequence Information

The genomic DNA sequence (SEQ ID NO: 20) of the 1 species of phage obtained was analyzed, resulting in revealing that the amino acid sequence encoded by the nucleotide sequence from the 32468th to the 34522nd (CDS) is a tail fiber protein. Using the amino acid sequence of this tail fiber protein as a query sequence, a search for a similar amino acid sequence and verification of the sequence identity therebetween were performed on the NCBI-provided BLAST server (https://blast.ncbi.nlm.nih.gov/Blast.cgi). As a result, the amino acid sequences of the tail fiber proteins of phages against many bacteria of the genus *Salmonella* were detected, but no amino acid sequence having a sequence identity of 100% was detected. Then, the amino acid sequences having a length of 684 residues, which is the same length of the tail fiber protein of the 6th phage, and having a sequence identity of 95% or more were extracted through search, and subjected to multiple alignment. The results are shown in Figure 12A, Figure 12B, and Figure 12C. Additionally, Table below shows information on: the names of the phages having a sequence used for alignment; the sequence identities; the Genbank access codes; the sequence ID numbers allocated herein; and the serotypes to which the phage reacts (with particular attention paid to Enteritidis and Typhimurium) that were confirmed from the registration information and the literature information.

**[Table 8]**

| *Salmonella* Phage Name | Identity (%) | GenBank Accession No. | SEQ ID NO | Serotype | | | |
|---|---|---|---|---|---|---|---|
| | | | | Enteritidis | Typhimurium | P | U |
| GSP003 | 98.1 | UXE05692.1 | 28 | | | ○ | |
| vB SenTO17 | 98.1 | YP 010582355.1 | 29 | × | ○ | | |
| TS6 | 97.81 | YP 010582256.1 | 30 | | ○ | | |
| LP31 | 97.51 | UGC97882.1 | 31 | | | ○ | |
| vB SenS SE1 | 97.37 | YP 010582462.1 | 32 | | × | | ○ |
| GRNsp6 | 97.37 | URG 17609.1 | 33 | ○ | ○ | | |
| PSDA-2 | 97.22 | QVW27666.1 | 34 | | ○ | | |
| vB SpuS Sp4 | 97.08 | AWY03030.1 | 35 | | | | ○ |
| CKT1 | 96.78 | UJP30002.1 | 36 | | | ○ | |
| nctD30 | 96.64 | USL89603.1 | 37 | | | | ○ |
| SHWT1 | 96.64 | QN120443.1 | 38 | Δ | ○ | ○ | |
| vB STM-ZS | 96.49 | YP 010582402.1 | 39 | | ○ | | |
| GRNsp27 | 96.35 | YP 010582173.1 | 40 | ○ | ○ | | |
| S55 | 96.05 | QMS41869.1 | 41 | ○ | | ○ | |

| | | | | | | | |
|---|---|---|---|---|---|---|---|
| >75%: ○, 75 to 50%: Δ, 50%>: ×, P: Pullorum, U: Unknown | | | | | | | |

As shown in Table 8, even with attention paid only to the 2 species Enteritidis and Typhimurium, it can be said that the host range of each phage is highly probably different although the sequence identity of the tail fiber protein is 95% or more.

Additionally, as understood from the results of alignment of the amino acid sequences, the tail fiber protein of the 6th phage has a plurality of unique amino acid residues different from all the other sequences. The residues are Val at the 211th (Ile in all the others), Val at the 321st (Ile in all the others), Val at the 485th (Ile or Met in others), Ala at the 533rd (Ser in all the others), Ser at the 577th (Gly in all the others), and Ser at the 583rd (Gly in all the others). It is a surprising fact that, although the amino acid residues at many sites are highly conserved in the tail fiber proteins of other phages, the amino acid residues in the 6th phage are different therefrom, as described above. The fact is conceivably in connection with the host range characteristic of the 6th phage.

Using the genomic DNA sequence of the 6th phage, a search of the BLAST server also brought about the result that the sequence having the highest sequence identity was *Salmonella* phage GRNsp27, and that the sequence identity was 94.64% (Cover 95%/Ident 99.62%). However, when both sequences were compared for similarity on MUMmer, using the gene analysis software GENETYX (https://www.genetyx.co.jp/), the result revealed that the identity of the range of from the 29733rd to the 34770th corresponding to the surrounding region of the tail fiber protein gene (the 32468th to the 34522nd) was low at 87% (see Table below). As expected, the result suggested that the 6th phage is a novel phage significantly different from a phage having a known gene region responsible for host recognition.

**[Table 9]**

| Start | End | Identity (%) | Reference Coverage (%) | Query Coverage (%) | Similarity Errors |
|---|---|---|---|---|---|
| 1 | 22531 | 99 | 55 | 54 | 113 |
| 22521 | 28331 | 96 | 14 | 14 | 217 |
| 29733 | 34770 | 87 | 12 | 12 | 642 |
| 35384 | 36603 | 97 | 2 | 2 | 31 |
| 37785 | 38379 | 85 | 1 | 1 | 85 |
| 38434 | 38582 | 85 | 0 | 0 | 22 |
| 39683 | 40784 | 98 | 2 | 2 | 13 |

### <Example 7: Isolation of 7th Bacteriophage, and Bacteriolytic Activity Thereof>

### (Purpose)

A novel bacteriophage having bacteriolytic activity against bacteria of the genus *Salmonella* is isolated, and its bacteriolytic activity against bacteria of the genus *Salmonella* is verified in this Example.

### (Method and Results)

### (1) Obtainment and Culture of Bacteria of the Salmonella Genus

The bacterial strains used in Example 7 are listed in Table below.

**[Table 10]**

| Strain ID | Serotype | Strain Name | Source of Supplv |
|---|---|---|---|
| SE11 | S. Entertidis | L-3080 | The National Institute of Animal Health, The National Agriculture and Food Research Organization |
| SE13 | S. Entertidis | L-3241 | The National Institute of Animal Health, The National Agriculture and Food Research Organization |
| SE14 | S. Entertidis | L-3244 | The National Institute of Animal Health, The National Agriculture and Food Research Organization |
| SE15 | *S.* Entertidis | L-3246 | The National Institute of Animal Health, The National Agriculture and Food Research Organization |
| SE16 | *S.* Entertidis | L-3247 | The National Institute of Animal Health, The National Agriculture and Food Research Organization |
| ST3 | *S.* Typhimurium | HRS-TST-219 | The Faculty of Veterinary Medicine, Rakuno Gakuen University |
| ST4 | *S.* Typhimurium | HRS-KST-31 | The Faculty of Veterinary Medicine, Rakuno Gakuen University |
| SI1 | *S.* Infantis | O7:Hr70A | The Faculty of Veterinary Medicine, Rakuno Gakuen University |
| SI2 | *S.* Infantis | O7:Hd70B | The Faculty of Veterinary Medicine, Rakuno Gakuen University |
| SI3 | *S.* Infantis | O7:Hr1.5 | The Faculty of Veterinary Medicine, Rakuno Gakuen University |
| SM | *S.* Montevideo | S. Montevideo No.1 | The Faculty of Veterinary Medicine, Rakuno Gakuen University |
| SJ | *S.* Javiana | L-750 | The Faculty of Veterinary Medicine, Rakuno Gakuen University |

### (2) Isolation and Purification of 7th Phage

In accordance with the method described in the section of [Isolation and Purification of Phage] above, a new phage (corresponding to the 7th phage) was isolated from natural dirty water and soil, and purified.

### (3) Amplification and Refinement of 7th Phage

In accordance with the method described in the section of [Amplification and Refinement of Phage] above, a refined solution of the 7th phage was prepared, and the titer was measured. The titer was confirmed to be 10⁸ PFU/mL or more.

### (4) Evaluation of Host Range of 7th Phage

In accordance with the method described in the section of [Evaluation of Host Range of Phage] above, the host range of the 7th phage was evaluated by a spot test method.

One example of the results is shown in Figure 10. The 7th phage obtained in the present Example exhibited bacteriolytic activity against S. Enteritidis, but did not exhibit bacteriolytic activity against any of S. Typhimurium, S. Infantis, S. Montevideo, and S. Javiana. S. Enteritidis is a serotype that is detected with the highest frequency in the food poisoning of a human (Oh and Park, *J. Microbiol. Biotechnol.* (2017), *27*(*12*)*,* 2075-2088). Accordingly, the 7th phage is particularly useful, for example, for treating or preventing food poisoning of a human. Additionally, the 7th phage exhibited bacteriolytic activity specifically against S. Enteritidis, and thus, is particularly useful for identifying S. Enteritidis.

### (5) Genomic Analysis of 7th Phage

The genomic DNA sequence of the 7th phage was determined and analyzed.

### (i) Preparation and Sequencing of Genomic DNA of 7th Phage

In accordance with the method described in the section of [Preparation and Sequencing of Genomic DNA of Phage] above, the genomic DNA sequence of the 7th phage was determined. The genomic DNA sequence determined of the 7th phage is shown in SEQ ID NO: 23.

### (ii) Bioinformatics Analysis Based on Genomic Sequence Information

Using the genomic DNA sequence of the 7th phage as a query sequence, a similar DNA sequence was searched for on the NCBI-provided BLAST server (https://blast.ncbi.nlm.nih.gov/Blast.cgi). As a result of the search, the nearest-neighbor nucleotide sequence was the genomic sequence of *Salmonella* phage SPN9CC (GenBank Accession No.: JF900176.1). According to Shin *et. al., Applied and Environmental Microbiology,* 2014, vol. *80,* No. *1*, 374-384, SPN9CC exhibits bacteriolytic activity against all of a total of 7 strains of S. Typhimurium. Accordingly, SPN9CC is obviously different in the host range from the 7th phage that specifically exhibits bacteriolytic activity against S. Enteritidis. Then, the amino acid sequence of a protein important for the host recognition was compared between the 7th phage and SPN9CC, with the result that a difference was found in the amino acid sequence of the tail spike protein. Accordingly, the result has revealed that the difference in the amino acid sequence of the tail spike protein is a cause for the difference in the host range between both phages. In this regard, a gene encoding the tail spike protein exists from the 30879th to 32882nd of the genomic DNA sequence of the 7th phage. The amino acid sequence of the tail spike protein of the 7th phage is shown in SEQ ID NO: 21, and the nucleotide sequence encoding the protein is shown in SEQ ID NO: 22.

In the search for the above-described similar DNA sequence, there was found no phage having a gene encoding a tail spike protein consisting of the amino acid sequence of SEQ ID NO: 21, and having a genomic DNA sequence having a sequence identity of 99% or more to the nucleotide sequence of SEQ ID NO: 23 in the whole range.

## Claims

1. A bacteriophage with bacteriolytic activity against bacteria of the genus *Salmonella,* wherein the bacteriophage has a genomic DNA comprising a gene encoding a tail tip protein having an ability to recognize target bacteria, and consisting of the amino acid sequence of any one of the following (a) to (c):
(a) the amino acid sequence of SEQ ID NO: 8;
(b) an amino acid sequence having addition, deletion, and/or substitution of one or a plurality of amino acids in the amino acid sequence of SEQ ID NO: 8; and
(c) an amino acid sequence having a sequence identity of 99% or more to the amino acid sequence of SEQ ID NO: 8.

2. The bacteriophage according to claim 1, wherein the gene encoding the tail tip protein comprises the nucleotide sequence of any one of the following (d) to (f):
(d) the nucleotide sequence of SEQ ID NO: 9;
(e) a nucleotide sequence having addition, deletion, and/or substitution of one or a plurality of nucleotides in the nucleotide sequence of SEQ ID NO: 9; and
(f) a nucleotide sequence having a sequence identity of 95% or more to the nucleotide sequence of SEQ ID NO: 9.

3. The bacteriophage according to claim 1, wherein the genomic DNA sequence comprises the nucleotide sequence of any one of the following (g) to (k):
(g) the nucleotide sequence of any one of SEQ ID NOs: 10 to 12;
(h) a nucleotide sequence having addition, deletion, and/or substitution of one or a plurality of nucleotides in the nucleotide sequence other than the nucleotide sequence of the gene within the nucleotide sequence of any one of SEQ ID NOs: 10 to 12;
(i) a nucleotide sequence having a sequence identity of 80% or more to the nucleotide sequence other than the nucleotide sequence of the gene within the nucleotide sequence of any one of SEQ ID NOs: 10 to 12;
(j) a nucleotide sequence having addition, deletion, and/or substitution of one or a plurality of nucleotides in the nucleotide sequence of any one of SEQ ID NOs: 10 to 12; and
(k) a nucleotide sequence having a sequence identity of 90% or more to the nucleotide sequence of any one of SEQ ID NOs: 10 to 12.

4. The bacteriophage according to claim 1, wherein the bacteria of the genus *Salmonella* are S. Enteritidis, S. Typhimurium, S. Infantis, S. Montevideo, and S. Javiana.

5. A composition comprising the bacteriophage according to claim 1.

6. The composition according to claim 5, for controlling at least S. Enteritidis, S. Typhimurium, S. Infantis, S. Montevideo, and S. Javiana.

7. The composition according to claim 5, wherein the composition is a pharmaceutical composition.

8. The composition according to claim 5, wherein the composition is a food/drink additive, a feed additive, or a drinking-water additive.

9. The composition according to claim 5, wherein the composition is food, drink, or feed.

10. The composition according to claim 5, wherein the composition is a cleaning agent, a disinfectant, a bactericide, or a sanitizer.

11. The composition according to claim 5, further comprising another bacteriophage(s) with bacteriolytic activity against bacteria of the *Salmonella.*

12. A method for controlling bacteria of the genus *Salmonella,* comprising:
a contacting step of contacting the bacteriophage according to claim 1 or the composition according to claim 5 to a subject of application.

13. A method for treating or preventing an infection caused by bacteria of the *Salmonella* in a subject, comprising:
an administering step of administering the bacteriophage according to claim 1 or the composition according to claim 5 to the subject.

14. A method for identifying a bacterium of the genus *Salmonella,* comprising:
a culturing step of culturing a subject bacterium isolated from a specimen suspected of comprising bacteria of the genus *Salmonella* to obtain a culture preparation;
a mixing step of mixing the culture preparation with the bacteriophage according to claim 1 to obtain a mixture;
a mixture culturing step of culturing the mixture under predetermined conditions; and
a determining step of determining that the subject bacterium is a bacterium of the *Salmonella,* when the subject bacterium is bacteriolyzed after the mixture culturing step.

15. The method according to claim 14, wherein, in the mixture culturing step, the mixture further comprises a soft agar containing liquid medium, and the mixture is cultured on a solid medium.

16. The method according to claim 14, wherein, in the culturing step, the culture preparation comprises a soft agar containing liquid medium, and the culture preparation is cultured on a solid medium.

17. The method according to claim 14, further comprising, before the culturing step, an isolating step of isolating the subject bacterium from the specimen suspected of comprising the bacteria of the genus *Salmonella.*
